# EUROPEAN PATENT SPECIFICATION

(11) **EP 3 232 733 B1**
(45) Date of publication and mention of the grant of the patent: **31.10.2018**
(21) Application number: 17165648.1
(22) Date of filing: 10.04.2017
(51) Int. Cl.: H05B 6/06, A47J 27/62, A61B 5/00, F24C 7/08, F24C 7/00, G01N 33/02, H05B 1/02, H05B 6/64

(54) **SYSTEM THAT EMITS LIGHT TO OVERHEATED PORTION OF COOKING CONTAINER**
SYSTEM MIT LICHTEMISSION AN ÜBERHITZTEN TEIL EINES KOCHBEHÄLTERS
SYSTÈME ÉMETTANT DE LA LUMIÈRE VERS LA PARTIE SURCHAUFFÉE D'UN RÉCIPIENT DE CUISSON

(30) Priority: 15.04.2016 JP 2016082431; 23.01.2017 JP 2017009849
(43) Date of publication of application: 18.10.2017
(73) Proprietor: Panasonic Intellectual Property Management Co., Ltd., Osaka-shi, Osaka 540-6207 (JP)
(72) Inventor: KAMEI, Rinako, Osaka-shi, Osaka 540-6207 (JP); RAFII, Zarina, Osaka-shi, Osaka 540-6207 (JP); ASO, Mitsuhiro, Osaka-shi, Osaka 540-6207 (JP); SUGIMOTO, Hiroko, Osaka-shi, Osaka 540-6207 (JP); YUKI, Yasuhiro, Osaka-shi, Osaka 540-6207 (JP)
(74) Representative: Grünecker Patent- und Rechtsanwälte PartG mbB

(56) References cited:
- WO-A1-2015/062666
- JP-A- 2014 025 721
- JP-A- 2015 068 542

## Description

### BACKGROUND

### 1. Technical Field

The present disclosure relates to a cooking support method and a cooking support system that support cooking by users.

### 2. Description of the Related Art

A cooking device such as an induction heating (IH) cooking heater and a microwave oven cooks food by heating. An IH cooking heater heats, for instance, a pot disposed on the top plate. Also, a microwave oven heats a cooking container such as a dish or a cup placed in the oven.

Although a cooking container is heated by such cooking devices, a user cannot easily determine whether or not the cooking container has been heated to a level that won't allow the user to hold it with a hand.

Meanwhile, a projection device has been proposed, that directly projects the temperature distribution of an object onto the object (see, for instance, Japanese Unexamined Patent Application Publication No. 2014-25721).

Using such a projection device allows the temperature distribution of a cooking container to be projected onto the cooking container.

However, there is a problem in that with the projection onto a cooking container by the projection device disclosed by Japanese Unexamined Patent Application Publication No. 2014-25721, it is difficult to notify a user of an overheated portion of the cooking container.

Specifically, a temperature distribution is projected onto the entire cooking container by the projection device disclosed by Japanese Unexamined Patent Application Publication No. 2014-25721, and thus a temperature distribution is projected onto even cooking ingredients in a cooking container. As a result, it is difficult for a user to visually determine whether or not the cooking ingredients have been cooked well. Furthermore, when a user transports a cooking container heated by the cooking device, the user holds the cooking container and projection of the temperature distribution onto an area other than a portion held by the user may cause confusion in the determination by the user as to the possibility of a burn. The present invention is defined by the claims. JP2015068542 discloses a method according to the preamble of claim 1.

### SUMMARY

One non-limiting and exemplary embodiment provides a cooking support method and a cooking support system that are capable of properly notifying a user of an overheated portion of a cooking container.

In one general aspect, the techniques disclosed here feature a cooking support method including: obtaining temperature-related information which is related to temperatures at portions of a cooking container, using a processor; recognizing an edge of the cooking container disposed in a cooking device that cooks by heating, and identifying an overheated portion in the edge where a temperature exceeds a threshold value based on the temperature-related information using the processor; and emitting light to the identified overheated portion by transmitting emission instruction information to a light emitter.

The cooking support method in the present disclosure allows a user to be properly notified of an overheated portion of a cooking container.

It should be noted that general or specific embodiments may be implemented as a system, a method, an integrated circuit, a computer program, a storage medium such as a computer readable CD-ROM, or any selective combination thereof.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

### BRIEF DESCRIPTION OF THE DRAWINGS

Fig. 1 is a configuration diagram of a cooking support system in a first embodiment;
Fig. 2 is a block diagram illustrating the configuration of a control device in the first embodiment;
Fig. 3 illustrates an example of an image obtained by capturing with a camera in the first embodiment;
Fig. 4 illustrates an example of an area of an edge of a cooking container in the first embodiment;
Fig. 5 illustrates an example of a temperature distribution indicated by temperature information in the first embodiment;
Fig. 6 is an illustration for explaining a timing at which light is emitted by a light emitter in the first embodiment;
Fig. 7 illustrates an example of the edge to which light is emitted by the light emitter in the first embodiment;
Fig. 8 is a flowchart illustrating the processing operation of the control device in the first embodiment;
Fig. 9 is a flowchart illustrating the processing operation of the control device in a first modification;
Fig. 10 illustrates an example of a temperature distribution indicated by temperature information in a second modification of the first embodiment;
Fig. 11 illustrates an example of an edge to which light is emitted by the light emitter in the second modification of the first embodiment;
Fig. 12 illustrates a cooking support system in a second embodiment;
Fig. 13 is a block diagram illustrating the configuration of a control device in the second embodiment;
Fig. 14 is a table illustrating an example of a determination rule stored in a memory in the second embodiment;
Fig. 15 illustrates an example in which light is emitted to a cooking container by the cooking support system in the second embodiment;
Fig. 16 illustrates another example in which light is emitted to a cooking container by the cooking support system in the second embodiment;
Fig. 17 is a flowchart illustrating the processing operation of the control device in the second embodiment;
Fig. 18 illustrates an example of the lateral surface of a cooking container to which light is emitted by a light emitter in a first modification of the second embodiment;
Fig. 19 illustrates an example of the lateral surface of a cooking container to which light is emitted from an upper position in a second modification of the second embodiment;
Fig. 20 is a block diagram illustrating an example of the configuration of a cooking support system in a third embodiment;
Fig. 21 is a block diagram illustrating another example of the configuration of the cooking support system in the third embodiment;
Fig. 22 is a flowchart illustrating the processing operation of a control device in the third embodiment;
Fig. 23A is a flowchart of a cooking support method according to an aspect of the present disclosure; and
Fig. 23B illustrates the configuration of a cooking support system according to an aspect of the present disclosure.

### DETAILED DESCRIPTION

An aspect of the present disclosure provides a method including: obtaining temperature-related information which is related to temperatures of portions of a cooking container, using a processor; recognizing an edge of the cooking container disposed in a cooking device that cooks by heating, and identifying an overheated portion in the edge where a temperature exceeds a threshold value, based on the temperature-related information using the processor; and transmitting emission instruction information to a light emitter, and causing the light emitter to emit light to the identified overheated portion. For instance, the method may further includes: obtaining a result of detection by a sensor that detects at least the temperatures of the portions of the cooking container, wherein, in the identifying, the edge of the cooking container is recognized, and the overheated portion is identified based on the result of detection.

Thus, the edge of the cooking container is recognized, and light is emitted to an overheated portion in the edge. Therefore, it is possible to allow a user to be properly notified of the overheated portion in the edge which is likely to be held by the user in the cooking container. Consequently, it is possible to reduce the occurrence of burn injuries due to contact of a bare hand with an overheated portion. Specifically, it is possible to reduce the risk of burn injuries due to contact of a bare hand with a highly heated cooking container such as a dish or a pot. Also, since light is not emitted to the cooking ingredients in the cooking container, it is possible to reduce the effect on the visual determination of a user as to whether or not the cooking ingredients have been cooked well.

For instance, the method may further includes: obtaining an image, by a camera, in a range including the cooking container disposed in the cooking device, wherein in the obtaining of the temperature information, the sensor is an infrared sensor, obtaining temperature information indicating the temperatures of the portions in the range, by the infrared sensor, wherein, in the identifying, the edge is recognized based on the image, and the overheating portion is identified based on the temperatures of the portions in the recognized edge out of the temperatures of the portions indicated by the temperature information.

Thus, the edge of the cooking container can be clearly recognized by the image of the camera, and an overheated portion in the edge can be accurately identified.

Also, the camera may capture the range from a position above the cooking container, and the light emitter, when receiving the emission instruction information, may emit light to the overheated portion in the edge from a position above the cooking container. Specifically, the cooking device may be an induction heating (IH) cooking heater.

When a user places a cooking container on an IH cooking heater for cooking, the user looks at the cooking container a position thereover and tends to hold the edge of the cooking container. Thus, in an aspect of the present disclosure, light is emitted to the overheated portion in the edge from a position above the cooking container, and it is possible to notify the user of the overheated portion in a comprehensible manner and to reduce the risk of burn injuries.

Also, the method may further includes: determining whether or not a bare human hand is approaching the overheated portion, based on the image; and when the light emitter is caused to emit light, transmitting the emission instruction information to the light emitter at a timing at which it is determined that the bare human hand is approaching the overheated portion.

Since light is emitted to the overheated portion when a bare human hand is approaching the overheated portion, a light emission period can be restricted to the time when the risk of burn injuries is increased. Thus, the power consumption of the cooking support system can be reduced.

Also, the method may further includes: determining whether or not a bare human hand is approaching the overheated portion based on the image obtained by the capturing with the camera when light is emitted to the overheated portion by the light emitter; and when it is determined that the bare human hand is approaching the overheated portion, performing processing to prompt warnings to people around the cooking device.

Thus, when a bare human hand is approaching the overheated portion during emission of light, processing to prompt warnings, for instance, processing to make alarm sound is performed, the risk of burn injuries can be more reduced.

Also, when the light emitter is caused to emit light, light of a color different from a color of the light emitted to the overheated portion may be emitted to any portion other than the overheated portion in the edge.

Consequently, light of a color different from the color of light for the overheated portion is emitted to an non-overheated portion which is a portion other than the overheated portion, and thus a user to be properly notified of, for instance, a portion which may be touched by a bare hand. Consequently, a user can save time and effort for preparing gloves such as a mitten.

Also, the method may further includes: obtaining a lateral surface image by a side-camera from a lateral side of the cooking container in the range; obtaining lateral surface temperature information on portions captured by the side-camera, by a side-infrared sensor; and determining a type of the cooking container based on the lateral surface image, wherein in the identifying, a set consisting of the image and the temperature information or a set consisting of the lateral surface image and the lateral surface temperature information is selected according to the determined type, and the overheated portion in the edge or the lateral surface of the cooking container is identified based on the selected set, wherein in the emitting of the light, when the overheated portion is in the lateral surface, light is emitted to the identified overheated portion by transmitting the emission instruction information to a side light emitter that emits light to the cooking container from the lateral side of the cooking container, instead of to the light emitter. Specifically, the cooking device may be a microwave oven.

A user puts a cooking container in a microwave oven for cooking, and when the cooking container is taken out, the user holds the lateral surface of the cooking container or the edge of the cooking container according to the type of the cooking container. For instance, when the cooking container is a cup or a glass, a user tends to hold the lateral surface of the cooking container, and when the cooking container is a dish or the like a user tends to hold the edge of the cooking container. Therefore, in an aspect of the present disclosure, an overheated portion in the edge or the lateral surface of the cooking container is identified according to the type of the cooking container and light is emitted to the overheated portion, and thus the risk of burn injuries can be reduced regardless of the type of the cooking container.

Also, the sensor is a thermal image sensor, and a thermal image indicating a temperature distribution in the range is obtained by the thermal image sensor, wherein in the identifying, the edge may be recognized based on the thermal image, and the overheated portion may be identified based on the temperature distribution in the recognized edge.

Consequently, a camera is not used to recognize the edge, and thus the configuration of the cooking support system can be simplified.

Also, the method may further includes: obtaining an image, by a camera, in a range including the cooking container placed in the cooking device, wherein in the identifying, the edge is recognized based on the image, and temperatures of the portions in the edge are estimated based on the temperature-related information, and the overheated portion is identified based on the estimated temperatures of the portions in the edge. For instance, the temperature-related information includes sensor information indicating part of temperatures of the cooking container which are detected by a sensor mounted in the cooking device.

Thus, even when the cooking support system is not equipped with a detection device that detects at least the temperature of each of the portions of the cooking container, the temperature of each portion in the edge of the cooking container is estimated, and an overheated portion is identified. Therefore, similarly to what has been described above, it is possible to allow a user to be properly notified of the overheated portion in the edge which is likely to be held by the user in the cooking container.

Also, the temperature-related information may further include operation setting information which is set to the cooking device for cooking using the cooking container. For instance, the operation setting information indicates at least one of a heating duration time, a set temperature and a mode for the cooking container.

Thus, the temperature of each of the portions in the edge of the cooking container can be accurately estimated.

Also, the temperature-related information may further include type information that indicates a type of the cooking container.

Thus, the temperature of each of the portions in the edge of the cooking container can be more accurately estimated.

Hereinafter, an embodiment will be specifically described with reference to the drawings.

It is to be noted that each embodiment described below represents a comprehensive or specific example. Numerical values, shapes, materials, components, arrangement positions and connection configurations of the components, steps, the order of the steps shown in the subsequent embodiments provide an example, and are not intended to limit the present disclosure. In addition, among the structural components in the subsequent embodiments, components not recited in any one of the independent claims which provide the most generic concepts are described as arbitrary structural components.

It is to be noted that the respective figures are schematic diagrams and are not necessarily precise illustrations. Furthermore, in the respective figures, the same reference sign is given to the same components.

### (First Embodiment)

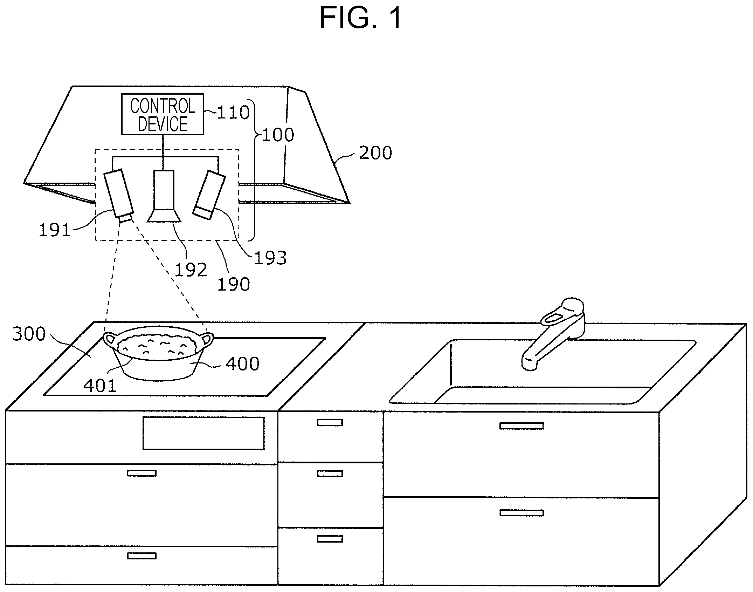

Fig. 1 is a configuration diagram of a cooking support system in a first embodiment.

A cooking support system 100 in this embodiment is disposed, for instance, in a range hood fan (hereinafter referred to as a range hood) 200 which is a ventilation fan for a kitchen, and supports heat cooking with an induction heating (IH) cooking heater 300 of a user. It is to be noted that although the cooking support system 100 is exposed from the range hood 200 in Fig. 1 in order to present the configuration of the cooking support system 100 in a comprehensible manner, the cooking support system 100 may be disposed inside the range hood 200. In other words, all or part of the cooking support system 100 may be disposed inside the range hood 200. Also, the IH cooking heater 300 is an example of a cooking device.

The cooking support system 100 includes a processing unit 190 that performs processing for supporting heat cooking, and control device 110 which controls the processing unit 190.

The processing unit 190 includes a detection device that detects at least the temperature of each of portions of a cooking container and outputs detection information indicating a result of the detection to the control device 110, and a light emitter 191 that emits light.

Here, the detection device in this embodiment includes, for instance, a camera 192 that captures a range including a heater formed on the top plate of the IH cooking heater 300, and an infrared sensor 193 that detects the temperature of each of the portions in the range. Specifically, infrared sensor 193 is a heat image sensor which detects the temperature distribution in the range.

The camera 192 outputs an image obtained by the capturing to the control device 110. The infrared sensor 193 outputs information indicating a temperature distribution obtained by the detection to the control device 110 as temperature information. The control device 110 obtains the information including such an image and temperature information as the detection information mentioned above.

Here, when a cooks by heating using the IH cooking heater 300, a cooking container 400 such as a pot with cooking ingredients is placed on the heater of the IH cooking heater 300. Therefore, at this point, the camera 192 captures a range including the cooking container 400 placed on the IH cooking heater 300 from a position above the cooking container 400, and outputs an image obtained by the capturing to the control device 110. It is to be noted that the "above" means "above in the vertical direction", and the cooking container 400 is placed on the heater of the IH cooking heater 300 with an opening of the cooking container 400 facing upward. Furthermore, the infrared sensor 193 detects the temperature of each of the portions in a range including the cooking container 400, and outputs temperature information indicating the temperature of each of the portions to the control device 110.

A light emitter 191 is, for instance, a projector and emits light of a predetermined color such as red according to the control from the control device 110.

The control device 110 includes of computers. Specifically, the control device 110 includes, for instance, a program executor such as a central processing unit (CPU) or a processor, and a recording medium such as a hard disk or a semiconductor memory. The program executor controls the processing unit 190 by reading and executing a software program recorded on the recording medium.

The control device 110 in this embodiment obtains detection information indicating a result of the detection by the above-described detection device from the detection device. The control device 110 then recognizes an edge 401 of the cooking container 400 placed on the IH cooking heater 300 that cooks food by heating, based on the detection information. In addition, the control device 110 identifies an overheated portion which is a portion where the temperature exceeds a threshold value in the edge 401. The control device 110 then transmits emission instruction information to the light emitter 191, thereby causing the light emitter 191 to emit light to the identified overheated portion. Specifically, the light emitter 191, when receiving the emission instruction information transmitted from the control device 110, emits light to the overheated portion in the edge 401 of the cooking container 400 from a position above the cooking container 400. It is to be noted that the above-mentioned threshold value is pre-set in the control device 110. For instance, the threshold value is 70 to 80°C. Alternatively, the threshold value may be changed by a setting operation of a user in any manner.

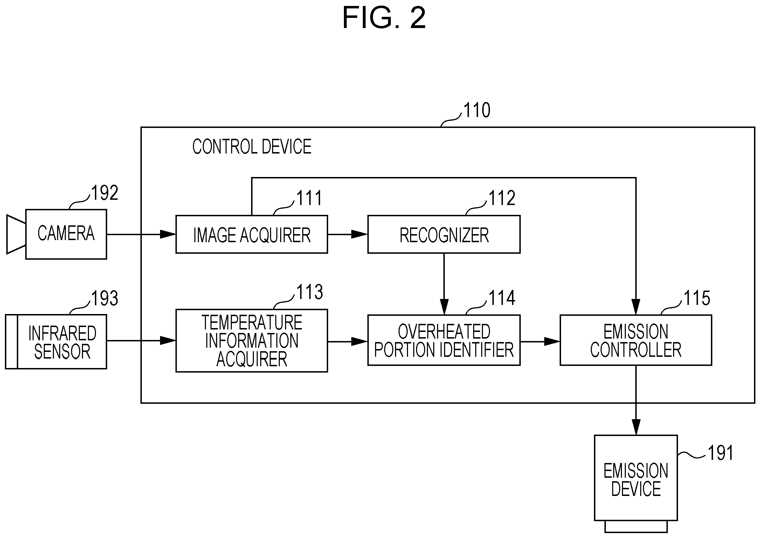

Fig. 2 is a block diagram illustrating the configuration of the control device 110.

The control device 110 includes an image acquirer 111, a recognizer 112, a temperature information acquirer 113, an overheated portion identifier 114 and an emission controller 115.

The image acquirer 111 obtains an image from the camera 192, and outputs the image to the recognizer 112. The recognizer 112 recognizes the edge 401 of the cooking container 400 based on the image, and outputs area information indicating an area of the recognized edge 401 to the overheated portion identifier 114.

The temperature information acquirer 113 obtains temperature information from the infrared sensor 193, and outputs the temperature information to the overheated portion identifier 114.

The overheated portion identifier 114 obtains the area information from the recognizer 112, and obtains the temperature information from the temperature information acquirer 113. The overheated portion identifier 114 then identifies an overheated portion if any based on the temperatures of portions in an area indicated by the area information out of the temperatures of the portions indicated by the temperature information. The overheated portion identifier 114 then outputs identification area information indicating the area of the identified overheated portion to the emission control unit 115.

The emission controller 115 transmits emission instruction information to the light emitter 191, the emission instruction information instructing that light is to be emitted to the area of overheated portion indicated by the identification area information outputted from the overheated portion identifier 114.

Upon receiving the emission instruction information, the light emitter 191 emits, for instance, a red light to the overheated portion in accordance with the emission instruction information.

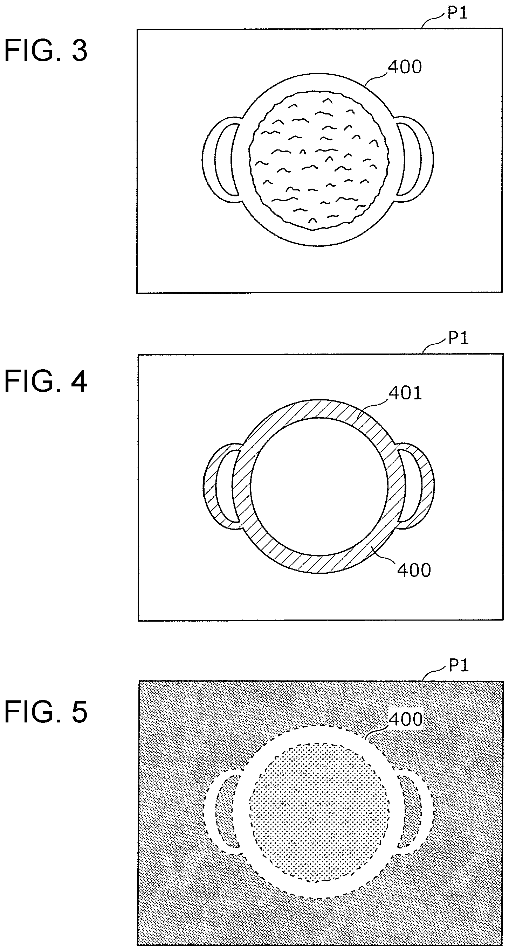

Fig. 3 illustrates an example of an image obtained by capturing with the camera 192.

The image acquirer 111 obtains an image P1 obtained by capturing a range including the cooking container 400 with the camera 192. In the image P1, for instance, the cooking container 400 with cooking ingredients appears. It is to be noted that the image P1 may be a static image or a dynamic image, and may be a color image or a monochrome image.

Fig. 4 illustrates an example of an area of the edge 401 of the cooking container 400.

The recognizer 112 recognizes the edge 401 of the cooking container 400 from the image P1. The recognizer 112 extracts the contour of the cooking container 400 by performing edge detection on the image P1, for instance, and recognizes the edge 401 that is an area having a width of a predetermined distance from the contour to the center side. Alternatively, the recognizer 112 may recognize the edge 401 by image processing such as pattern matching. The edge 401 is the periphery of the opening of a cooking container such as a pot, a dish, or a frying pan, and when a handle is attached to the periphery, the edge 401 includes the periphery and the handle. The recognizer 112 outputs the area information indicating the area of thus recognized edge 401 to the overheated portion identifier 114.

Fig. 5 illustrates an example of a temperature distribution indicated by temperature information.

The temperature information acquirer 113 obtains temperature information that indicates a temperature distribution. The temperature information is specifically a thermal image. For instance, in a thermal image, the color of the periphery of the cooking container 400 indicates a low temperature, the color of the cooking container 400 indicates a high temperature, and the color of the cooking ingredients in the cooking container 400 indicates a temperature lower than the cooking container 400 and higher than the periphery of the cooking container 400.

The overheated portion identifier 114 identifies the temperature distribution in the area of the edge 401 illustrated in Fig. 4 from the temperature distribution illustrated in Fig. 5, and further identifies an overheated portion in to the area if any, which is a portion where the temperature exceeds a threshold value. That is, the overheated portion identifier 114 identifies an overheated portion in any based on the temperatures of the portions in the recognized edge 401 out of the temperatures of the portions indicated by the temperature information.

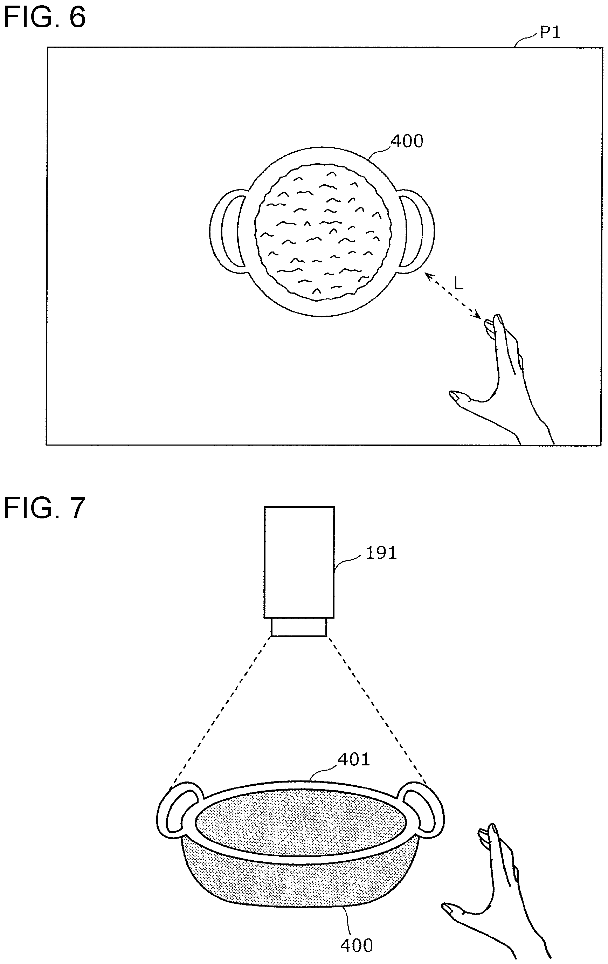

Fig. 6 is an illustration for explaining a timing at which light is emitted by the light emitter 191.

The emission controller 115 obtains an image P1 from the camera 192 via the image acquirer 111. The emission controller 115, when recognizing a bare human hand from the image P1, transmits the above-mentioned emission instruction information to the light emitter 191. Alternatively, when a distance L between the overheated portion of cooking container 400 appears in the image P1 and the bare human hand is less than or equal to a predetermined emission distance, the emission controller 115 transmits the emission instruction information to the light emitter 191. In this manner, the emission controller 115 determines based on the image P1 whether or not a bare human hand is approaching the overheated portion, and transmits the emission instruction information to the light emitter 191 at a timing at which it is determined that a bare human hand is approaching the overheated portion. Thus, when a bare human hand approaches the overheated portion, for instance, a red light is emitted to the overheated portion. It is to be noted that the light emitter 191 recognizes a bare human hand from the image P1 by image processing such as edge detection and pattern matching. As described above, the emission controller 115 may determine whether or not a bare human hand is approaching the overheated portion, or may determine whether or not a bare human hand is approaching the edge 401. Alternatively, the emission controller 115 may determine whether or not a bare human hand is approaching the cooking container 400.

Fig. 7 illustrates an example of the edge 401 to which light is emitted by the light emitter 191.

For instance, when identifying each of all portions in the edge 401 as an overheated portion, the overheated portion identifier 114 outputs identification area information indicating the entire area of the edge 401 to the emission controller 115. Upon obtaining such identification area information, the emission controller 115 transmits the emission instruction information to the light emitter 191, thereby causing the light emitter 191 to emit, for instance, a red light to the entirety of the edge 401. At this point, the light emitter 191 emits a red light only to the edge 401 of the cooking container 400, and does not emit light to any portion other than the edge 401.

When a red light is emitted to the entire edge 401 in this manner, a user can easily tell that any portion in the edge 401 is overheated, and can avoid holding the edge 401 with a bare hand.

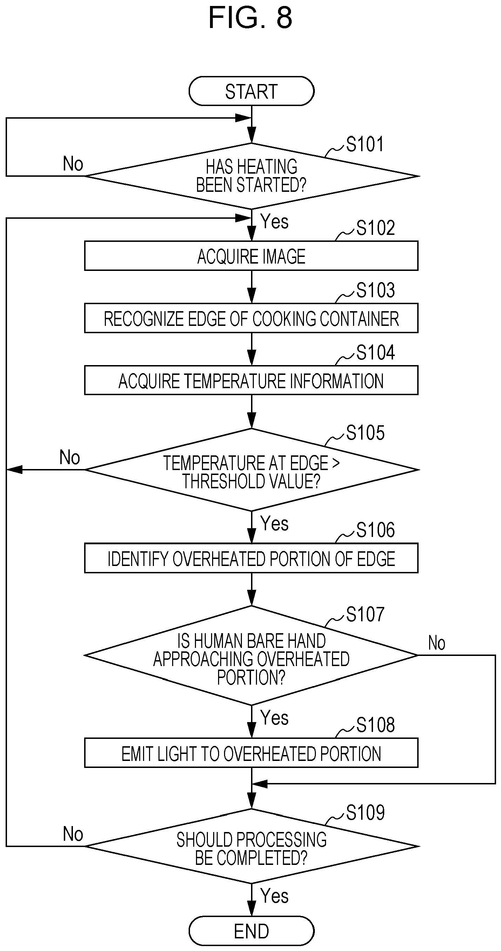

Fig. 8 is a flowchart illustrating the processing operation of the control device 110.

First, the control device 110 determines whether or not heating by the IH cooking heater 300 has started (step S101). Here, when it is determined that heating has started (Yes in step S101), the image acquirer 111 of the control device 110 obtains an image P1 from the camera 192 (step S102).

Next, the recognizer 112 recognizes the edge 401 of the cooking container 400 from the image P1 (step S103).

Subsequently, the overheated portion identifier 114 obtains temperature information from the infrared sensor 193 via the temperature information acquirer 113 (step S104). The overheated portion identifier 114 then determines whether or not the temperature in the recognized edge 401 exceeds a threshold value (step S105).

Here, when it is determined that the temperature in the edge 401 does not exceed a threshold value (No in step S105), the control device 110 performs the processing in and after step S102 repeatedly. On the other hand, when it is determined that the temperature in the edge 401 exceeds a threshold value (Yes in step S105), the overheated portion identifier 114 identifies an overheated portion from the edge 401 (step S106).

Subsequently, the emission controller 115 determines whether or not a bare human hand is approaching the overheated portion (or the cooking container 400) based on the image P1 (step S107). Here, when it is determined that a bare human hand is approaching (Yes in step S107), the emission controller 115 transmits the emission instruction information to the light emitter 191. Consequently, the light emitter 191 emits a red light to the overheated portion in the edge 401 of the cooking container 400 (step S108).

The control device 110 then determines whether or not the processing should be completed (step S109), and when it is determined that the processing should not be completed (No in step S109), the processing in and after step S102 is performed repeatedly. For instance, the control device 110 determines that the processing should be completed when a predetermined time has elapsed since heating by the IH cooking heater 300 has completed, or when the cooking container 400 is transported from the IH cooking heater 300.

### [Summary and Effects of First Embodiment]

As described above, the first embodiment provides a cooking support method in the cooking support system 100 that supports cooking of a user. The cooking support system 100 has the detection device that detects at least the temperature of each of the portions of the cooking container, the light emitter 191 that emits light, and the control device 110 that is a computer. The detection device includes a camera 192 that captures a range including the cooking container 400 placed on the IH cooking heater 300, and the infrared sensor 193 that detects the temperature of each of the portions in the range. Also, the camera 192 captures a range including the cooking container 400 from a position above the cooking container 400.

In the cooking support method in the first embodiment, the control device 110 first obtains detection information indicating a result of the detection by the detection device from the detection device. Specifically, the control device 110 obtains detection information including an image obtained by capturing with the camera 192, and the temperature information indicating the temperatures of the portions of the above-mentioned range detected by the infrared sensor 193. Subsequently, the control device 110 recognizes the edge 401 of the cooking container 400 placed on the IH cooking heater 300 that cooks by heating based on the detection information, and identifies an overheated portion if any which is a portion where the temperature exceeds a threshold value in the edge 401. Specifically, the control device 110 recognizes the edge 401 based on the above-mentioned image, and identifies an overheated portion in any based on the temperatures of the portions in the recognized edge 401 out of the temperatures of the portions indicated by the above-mentioned temperature information, The control device 110 then transmits emission instruction information to the light emitter 191, thereby causing the light emitter 191 to emit light to the identified overheated portion. The light emitter 191, when receiving the emission instruction information transmitted from the control device 110, emits light to the overheated portion in the edge 401 from a position above the cooking container 400.

In this manner, the edge 401 of the cooking container 400 is recognized and light is emitted to the overheated portion in the edge 401. Therefore, it is possible to allow a user to be properly notified of the overheated portion in the edge 401 which is likely to be held by the user in the cooking container 400. Consequently, it is possible to reduce the occurrence of burn injuries due to contact of a bare hand with an overheated portion. Specifically, it is possible to reduce the risk of burn injuries due to contact of a bare hand with a highly heated cooking container such as a dish or a pot. Also, since light is not emitted to the cooking ingredients in the cooking container 400, it is possible to reduce the effect on the visual determination of a user as to whether or not the cooking ingredients have been cooked well.

### [First Modification of First Embodiment]

In the first embodiment, when a user bare hand approaches an overheated portion, the cooking support system 100 emits light to the overheated portion. On the other hand, the cooking support system 100 in a first modification emits light to the overheated portion regardless of approach of a bare human hand to the overheated portion.

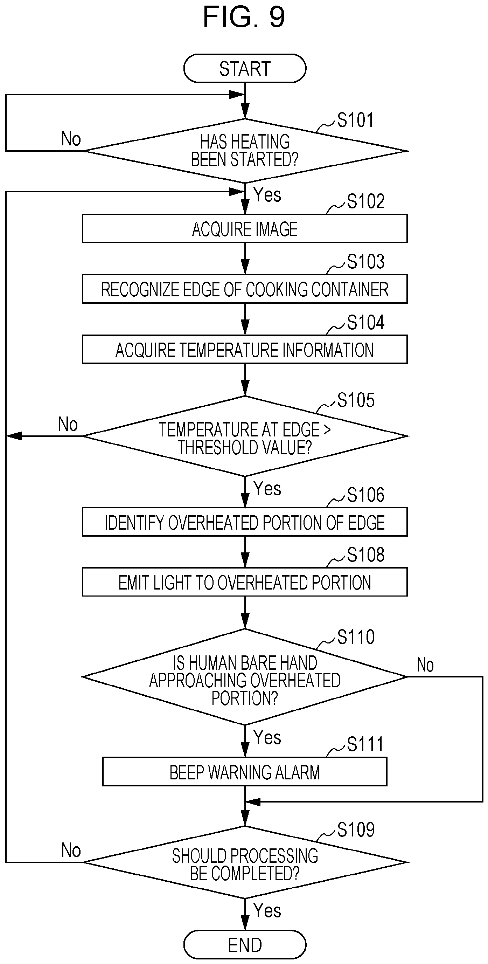

Fig. 9 is a flowchart illustrating the processing operation of the control device 110 in the first modification.

The control device 110 in the first modification does not perform the processing of step S107 in the flowchart illustrated in Fig. 8 but performs the processing in step S101 to S106 and S108. In other words, in the first modification, when an overheated portion is identified in the edge 401, the control device 110 causes the light emitter 191 to emit light to the overheated portion regardless of approach of a bare human hand to the overheated portion.

Thus, when an overheated portion is present, a user can prepare gloves such as a mitten well beforehand, and for instance, when the cooking container is going to be held, the user does not have to search for gloves in a hurry.

Also, during emission of light to an overheated portion in step S108, the control device 110 in the first modification determines whether or not a bare human hand is approaching the overheated portion (step S110). For instance, the emission controller 115 obtains an image (hereinafter referred to as a subsequent image) at an image capture time after the image capture time of the image P1 obtained in step S102, from the camera 192 via the image acquirer 111. When a bare human hand, which has not been recognized from the image P1, is recognized in the subsequent image, the emission controller 115 determines that a bare human hand is approaching the overheated portion. Alternatively, the distance between the overheated portion of the cooking container 400 and the bare human hand appearing in the subsequent image is determined to be shorter than the distance between the overheated portion of the cooking container 400 and the bare human hand appearing in image P1, the emission controller 115 determines that a bare human hand is approaching the overheated portion. It is to be noted that as described above, the emission controller 115 may determine whether or not a bare human hand is approaching the overheated portion or may determine whether or not a bare human hand is approaching the edge 401. Alternatively, the emission controller 115 may determine whether or not a bare human hand is approaching the cooking container 400.

When it is determined that a bare human hand is approaching (Yes in step S110), the emission controller 115 outputs alarm sound from a speaker (not illustrated) provided in the cooking support system (step S111). In other words, when it is determined that a bare human hand is approaching the overheated portion, the emission controller 115 performs processing to prompt warnings to the people around the IH cooking heater 300. It is to be noted that although the emission controller 115 outputs alarm sound as the processing to prompt warnings in the above-described example, the emission controller 115 may blink the light emitted to the overheated portion or may change the color of the light.

Subsequently, the control device 110 determines whether or not the processing should be completed (step S109), and when it is determined that the processing should not be completed (No in step S109), the processing in and after step S102 is performed repeatedly.

In this manner, in the cooking support method in the first modification, while the light emitter 191 emits light to an overheated portion, the control device 110 determines whether or not a bare human hand is approaching the overheated portion based on an image obtained by capturing with the camera 192. When it is determined that a bare human hand is approaching the overheated portion, the control device 110 performs processing to prompt warnings to the people around the IH cooking heater 300.

Thus, the risk of burn injuries can be more reduced.

### [Second Modification of First Embodiment]

In the first embodiment, the cooking support system 100 emits light to an overheated portion in the edge 401 of the cooking container 400, and does not emit light to any portion (that is, non-overheated portion) other than the overheated portion. In contrast, the cooking support system 100 in the second modification emits light of a color different from the color of the light emitted to the overheated portion to a non-overheated portion in the edge 401.

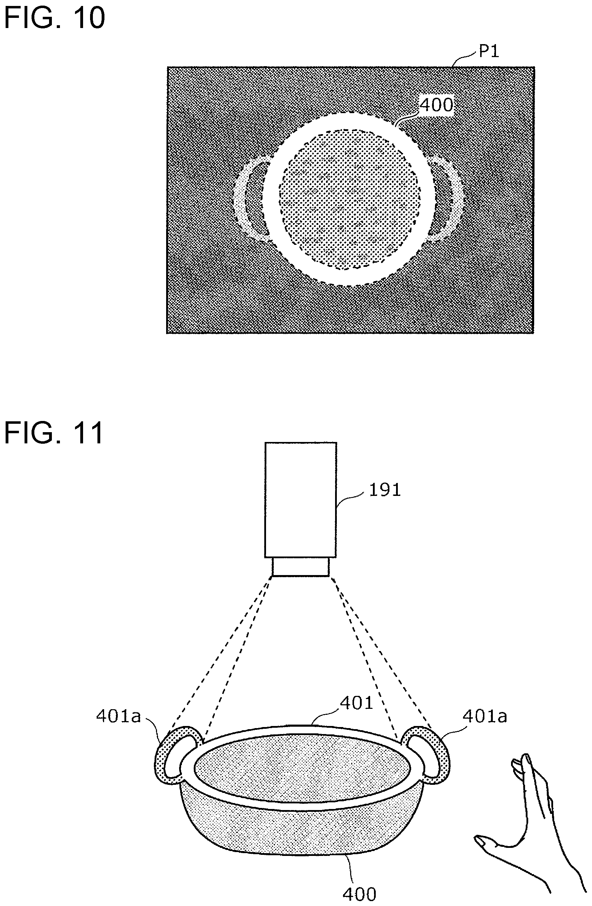

Fig. 10 illustrates an example of a temperature distribution indicated by temperature information.

For instance, in the temperature information obtained by the temperature information acquirer 113, that is, in a thermal image, the color of the periphery of the cooking container 400 and the color of each handle included in the edge 401 of the cooking container 400 both indicate a low temperature. In this case, the overheated portion identifier 114 identifies any portion other than the handle in the edge 401 as an overheated portion. The overheated portion identifier 114 then outputs identification area information indicating the area of the edge 401 and the area of the overheated portion to the emission controller 115.

Fig. 11 illustrates an example of the edge 401 to which light is emitted by the light emitter 191.

The emission controller 115, when obtaining the identification area information outputted as described above, transmits emission instruction information to the light emitter 191. The emission instruction information is information instructing that for instance, a red light is to be emitted to the area of the overheated portion indicated by the identification area information, and, for instance, a green light is to be emitted to the area other than the area of the overheated portion out of the area of the edge 401 indicated by the identification area information.

When receiving such emission designation information, as illustrated in Fig. 11, the light emitter 191 emits, for instance, a red light to the overheated portion other than handles 401a of the edge 401, and emits, for instance, a green light to the non-overheated portions which are the handles 401a of the edge 401.

In this manner, in the second modification, when the light emitter 191 is caused to emit light, the control device 110 causes the light emitter 191 to emit light of a color different from the color of the light emitted to the overheated portion to any portion other than the overheated portion in the edge 401.

This allows a user to be properly notified of the overheated portion in the edge 401, as well as of the non-overheated portion (for instance, the handle 401a) in the edge 401. That is, a user can be properly notified of a portion which may be touched by a bare hand. Consequently, a user can save time and effort for preparing gloves such as a mitten.

### (Second Embodiment)

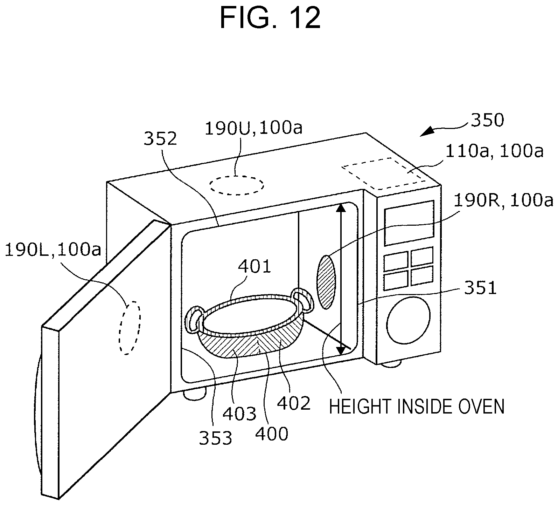

Fig. 12 illustrates a cooking support system in a second embodiment.

A cooking support system 100a in this embodiment is placed, for instance, in a microwave oven 350, and supports heat cooking by a user with the microwave oven 350. It is to be noted that the microwave oven 350 is an example of the cooking device.

The cooking support system 100a in this embodiment includes three processing units 190R, 190U, 190L that perform processing for supporting heat cooking, and the control device 110a that controls those three processing units 190R, 190U, 190L.

Similarly to the processing unit 190 in the first embodiment, each of the processing units 190R, 190U, 190L includes the light emitter 191, the camera 192, and the infrared sensor 193. The processing unit 190R is disposed in a right-side wall 351 in the chamber of the microwave oven 350, the processing unit 190U is disposed in an upper wall 352 in the chamber of the microwave oven 350, and the processing unit 190L is disposed in a left-side wall 353 in the chamber of the microwave oven 350. It is to be noted that in this embodiment, in opposite directions of the right-side wall 351 and the left-side wall 353, the direction of the right-side wall 351 is referred to as the right, the right direction, or the right side, and the direction of the left-side wall 353 in the opposite directions is referred to as left, left direction or left side. The direction outwardly of the microwave oven 350 in a direction perpendicular to the upper wall 352 is referred to as up, upward, upper direction or upper side.

It is to be noted that hereinafter when the respective light emitters 191 provided the processing units 190R, 190U, 190L are distinguished, those light emitters 191 are denoted by the light emitters 191R, 191U, 191L, respectively. Specifically, the light emitter 191R is the light emitter 191 provided in the processing unit 190R, the light emitter 191U is the light emitter 191 provided in the processing unit 190U, and the light emitter 191L is the light emitter 191 provided in the processing unit 190L. Similarly, when the respective cameras 192 provided in the processing units 190R, 190U, 190L are distinguished, those cameras 192 are denoted by the cameras 192R, 192U, 192L, respectively. Similarly, when the respective infrared sensors 193 provided in the processing units 190R, 190U, 190L are distinguished, those infrared sensors 193 are denoted by the infrared sensors 193R, 193U, 193L, respectively.

For instance, as an example of a cooking container, the cooking container 400 such as a pot is placed in the chamber of the microwave oven 350.

In this case, the camera 192R captures a range including the cooking container 400 placed in the chamber of the microwave oven 350 from the right-side wall 351. Specifically, the camera 192R captures a range including a right lateral surface 402 of the cooking container 400 as viewed from the right-side wall 351. The infrared sensor 193R detects the temperature of each portion in a range including the right lateral surface 402 of the cooking container 400, and outputs temperature information indicating a result of the detection to the control device 110a. The light emitter 191R emits light to the cooking container 400 from the right side of the cooking container 400.

The camera 192U captures a range including the cooking container 400 placed in the chamber of the microwave oven 350 from the upper wall 352. Specifically, the camera 192U captures a range including the cooking container 400 as viewed from the upper wall 352. The infrared sensor 193U detects the temperature of each portion in a range including the cooking container 400, and outputs temperature information indicating a result of the detection to the control device 110a. The light emitter 191U emits light to the cooking container 400 from the upper side of the cooking container 400.

The camera 192L captures a range including the cooking container 400 placed in the chamber of the microwave oven 350 from the left-side wall 353. Specifically, the camera 192L captures a range including a left lateral surface 403 of the cooking container 400 as viewed from the left-side wall 353. The infrared sensor 193L detects the temperature of each portion in a range including the left lateral surface 403 of the cooking container 400, and outputs temperature information indicating a result of the detection to the control device 110a. The light emitter 191L emits light to the cooking container 400 from the left side of the cooking container 400.

When the shape of the cooking container 400 as viewed from the upper wall 352 of the microwave oven 350 is approximately circular, the entire lateral surface of the cooking container 400 is divided into the right lateral surface 402 and the left lateral surface 403. In other words, the front surface of the cooking container 400 consists of the left side area of the right lateral surface 402 and the right side area of the left lateral surface 403. It is to be noted that the front surface of the cooking container 400 is the surface of the cooking container 400 when the cooking container 400 placed in the chamber of the microwave oven 350 is viewed from the opening side of the microwave oven 350.

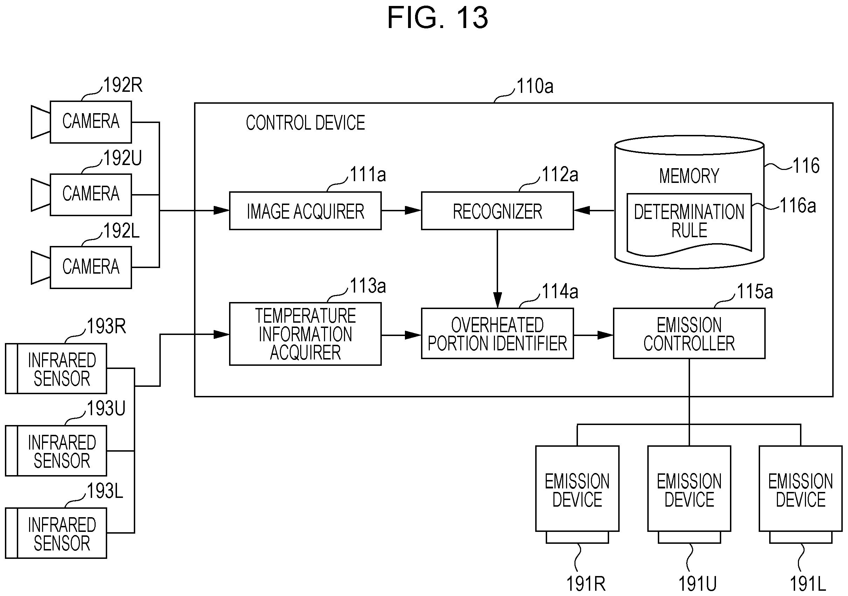

Fig. 13 is a block diagram illustrating the configuration of the control device 110a.

The control device 110a includes an image acquirer 111a, a recognizer 112a, a temperature information acquirer 113a, an overheated portion identifier 114a, an emission controller 115a, and a memory 116.

The image acquirer 111a obtains an image of a cooking container from the cameras 192R, 192U and 192L, and outputs the image to the recognizer 112a.

The memory 116 stores a determination rule 116a that is a rule for determining the type (container type) of the cooking container.

The recognizer 112a determines the container type of the cooking container placed in the chamber of the microwave oven 350 based on an image obtained from at least one of the cameras 192R and 192L via the image acquirer 111a. At this point, the recognizer 112a determines the container type of the cooking container appearing in the image in accordance with the determination rule 116a stored in the memory 116. In addition, the recognizer 112a recognizes the edge or the lateral surface (the right lateral surface 402 and the left lateral surface 403) of the cooking container based on a result of the determination. The recognizer 112a then outputs area information indicating the recognized edge or the area of the lateral surface to the overheated portion identifier 114a.

The temperature information acquirer 113a obtains temperature information from the infrared sensors 193R, 193U and 193L, and outputs the temperature information to the overheated portion identifier 114a.

The overheated portion identifier 114a obtains area information from the recognizer 112a, and obtains temperature information from the temperature information acquirer 113. The overheated portion identifier 114a then identifies an overheated portion based on the temperatures of portions in the area indicated by the area information out of the temperatures of portions indicated by the temperature information. The overheated portion identifier 114a then outputs identification area information indicating the area of the identified overheated portion to the emission controller 115a.

The emission controller 115a transmits emission instruction information to the light emitters 191R, 191U or 191L, the emission instruction information instructing that light is to be emitted to the area of the overheated portion indicated by the identification area information outputted from the overheated portion identifier 114a.

The control device 110a obtains detection information including the upper surface image which is an image obtained by capturing with the camera 192U, the lateral surface image which is an image obtained by capturing with the cameras 192R and 192L (side cameras), the upper surface temperature information which is temperature information obtained by detection with the infrared sensor 193U, and the lateral surface temperature information which is temperature information obtained by detection with the infrared sensors 193R and 193L (side infrared sensors).

The control device 110a determines the container type based on the lateral surface image. When an overheated portion is identified, the control device 110a selects a set consisting of the upper surface image and the upper surface temperature information or a set consisting of the lateral surface image and the lateral surface temperature information according to the determined container type. Subsequently, the control device 110a identifies an overheated portion in the edge or the lateral surface of the cooking container based on the selected set. Specifically, when the container type is shallow, the control device 110a selects a set consisting of the upper surface image and the upper surface temperature information, and similarly to the first embodiment, identifies an overheated portion in the edge of the cooking container. On the other hand, when the container type is deep, the control device 110a selects a set consisting of the lateral surface image and the lateral surface temperature information, and identifies an overheated portion in the lateral surface (the right lateral surface and the left lateral surface) of the cooking container. When the overheated portion is in the lateral surface, the control device 110a transmits emission instruction information to the light emitters 191R and 191L (side light emitters) instead of the light emitter 191U, thereby causing the light emitters 191R and 191L to emit light to the identified overheated portion. Also when the overheated portion is in the edge, the control device 110a transmits emission instruction information to the light emitter 191U, thereby causing the light emitter 191U to emit light to the identified overheated portion.

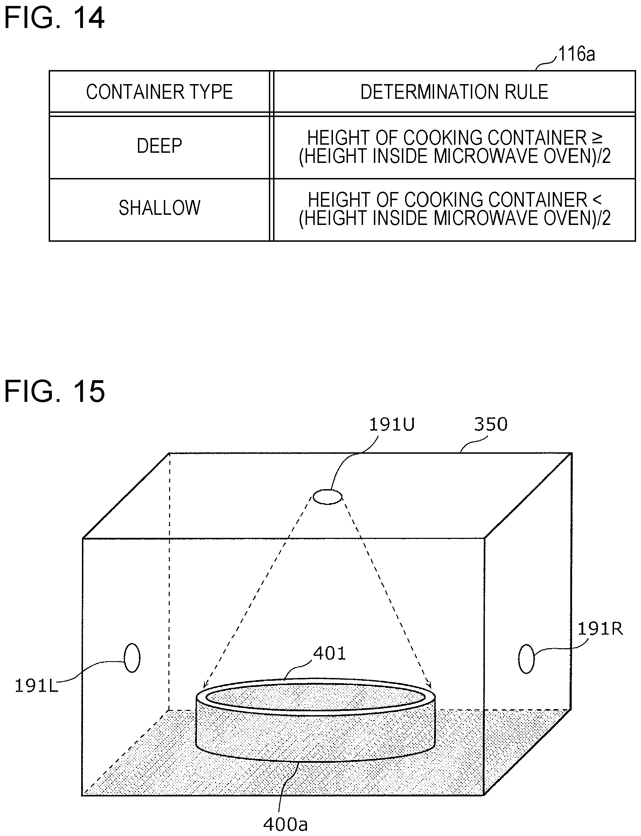

Fig. 14 is a table illustrating an example of the determination rule 16a stored in the memory 116.

The determination rule 116a indicates a determination criteria corresponding to each of multiple cooking containers (container types), and multiple container types. For instance, the determination rule 116a indicates deep or shallow as the container type. As the determination criteria corresponding to the container type "deep", the determination rule 116a indicates that the height of the cooking container is greater than or equal to one half of the height of the chamber of the microwave oven 350. Furthermore, as the determination criteria corresponding to the container type "shallow", the determination rule 116a indicates that the height of the cooking container is less than one half of the height of the chamber of the microwave oven 350. A cooking container of the container type "deep" is, for instance, a cup, a glass or a bowl, and for such a cooking container, it is highly probable that the lateral surface is held by a user. On the other hand, a cooking container of the container type "shallow" is, for instance, a pot, a frying pan or a dish, and for such a cooking container, it is highly probable that the edge (including a handle) is held by a user.

Fig. 15 illustrates an example in which light is emitted to a cooking container by the cooking support system 100a.

For instance, as an example of a cooking container, a cooking container 400a such as a dish is placed in the chamber of the microwave oven 350.

In this case, the recognizer 112a identifies the height of the cooking container 400a appearing in the image obtained by capturing with the cameras 192R and 192L, and compares the height of the cooking container 400a with one half of the height of the chamber. It is to be noted that the recognizer 112a may store one half of the height of the chamber of the microwave oven 350, or may read one half of the height from a recording medium such as a memory. The recognizer 112a then refers to the determination rule 116a stored in the memory 116, and determines that the container type of the cooking container 400a is "shallow" because the height of the cooking container 400a is less than one half of the height of the chamber.

Thus, similarly to the first embodiment, the recognizer 112a recognizes the edge 401 of the cooking container 400a from an image obtained by capturing of a range including the cooking container 400a via the camera 192U out of the cameras 192R, 192U and 192L. The recognizer 112a then outputs arrangement information to the overheated portion identifier 114a, the arrangement information indicating area information that indicates the area of the recognized edge 401, and the position (the upper wall 352) at which the camera 192U is disposed.

The overheated portion identifier 114a obtains area information and arrangement information from the recognizer 112a. The overheated portion identifier 114a obtains temperature information via the temperature information acquirer 113a, the temperature information being outputted from the infrared sensor 193U disposed at the position (the upper wall 352) indicated by the arrangement information. The overheated portion identifier 114a identifies an overheated portion in the edge 401 based on the temperatures of portions in the area indicated by the area information out of the temperatures of portions indicated by the temperature information. The overheated portion identifier 114a outputs identification area information indicating the area of the identified overheated portion and the above-described arrangement information to the emission controller 115a.

The emission controller 115a obtains the identification area information and the arrangement information from the overheated portion identifier 114a. Out of the light emitters 191R, 191U and 191L, the emission controller 115a selects the light emitter 191U disposed at the position (the upper wall 352) indicated by the arrangement information. The emission controller 115a then transmits emission instruction information to the light emitter 191U, the emission instruction information instructing that light is to be emitted to the area of the overheated portion indicated by the identification area information.

Upon receiving the emission instruction information, the light emitter 191U emits light to the overheated portion in the edge 401 of the cooking container 400a in accordance with the emission instruction information. For instance, when the area of the identified overheated portion is the entire edge 401, the light emitter 191U emits a red light to the entire edge 401.

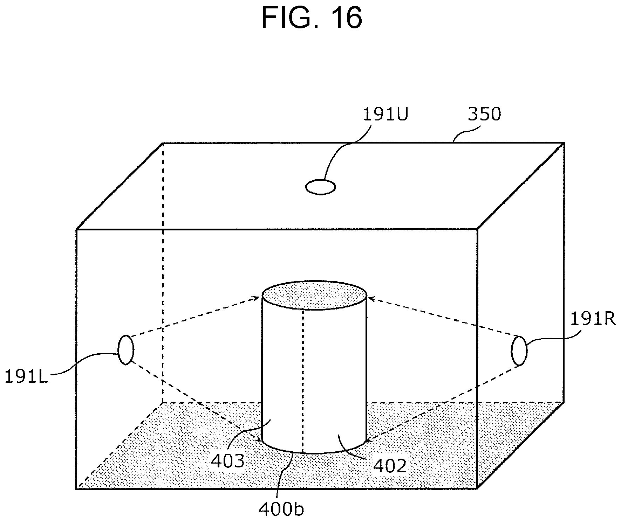

Fig. 16 illustrates another example in which light is emitted to the cooking container by the cooking support system 100a.

For instance, as an example of a cooking container, a cooking container 400b such as a cup or a glass is placed in the chamber of the microwave oven 350.

In this case, the recognizer 112a identifies the height of the cooking container 400b appearing in the image obtained by capturing with the cameras 192R and 192L, and compares the height of the cooking container 400b with one half of the height of the chamber. The recognizer 112a then refers to the determination rule 116a stored in the memory 116, and determines that the container type of the cooking container 400b is "deep" because the height of the cooking container 400b is greater than or equal to one half of the height of the chamber.

Thus, the recognizer 112a recognizes the right lateral surface 402 and the left lateral surface 403 of the cooking container 400a from an image obtained by capturing a range including the cooking container 400b with the cameras 192R and 192L out of the cameras 192R, 192U and 192L. The recognizer 112a then outputs area information and arrangement information to the overheated portion identifier 114a, the area information indicating the area of the recognized right lateral surface 402 and left lateral surface 403, the arrangement information indicating the positions (the right-side wall 351 and the left-side wall 353) at which the cameras 192R and 192L are disposed.

The overheated portion identifier 114a obtains area information and arrangement information from the recognizer 112a. The overheated portion identifier 114a obtains temperature information via the temperature information acquirer 113a, the temperature information being outputted from each of the infrared sensors 193R and 193L disposed at the positions (the right-side wall 351 and the left-side wall 353) indicated by the arrangement information. The overheated portion identifier 114a then identifies an overheated portion in the right lateral surface 402 and an overheated portion in the left lateral surface 403 based on the temperatures of portions in the area indicated by the area information out of the temperatures of portions indicated by the temperature information. The overheated portion identifier 114a outputs the identification area information indicating the areas of those identified overheated portions, and the above-described arrangement information to the emission controller 115a.

The emission controller 115a obtains the identification area information and the arrangement information from the overheated portion identifier 114a. Out of the light emitters 191R, 191U and 191L, the emission controller 115a selects the light emitters 191R and 191L disposed at the positions (the right-side wall 351 and the left-side wall 353) indicated by the arrangement information. The emission controller 115a then transmits emission instruction information to the light emitter 191R, the emission instruction information instructing that light is to be emitted to the overheated portion in the right lateral surface 402 indicated by the identification area information. In addition, the emission controller 115a transmits emission instruction information to the light emitter 191L, the emission instruction information instructing that light is to be emitted to the overheated portion in the left lateral surface 403 indicated by the identification area information.

Upon receiving the emission instruction information, the light emitter 191R emits light to the overheated portion in the right lateral surface 402 in accordance with the emission instruction information. For instance, when the identified overheated portion is the entire right lateral surface 402, the light emitter 191R emits a red light to the entire right lateral surface 402.

Upon receiving the emission instruction information, the light emitter 191L emits light to the overheated portion in the left lateral surface 403 in accordance with the emission instruction information. For instance, when the identified overheated portion is the entire left lateral surface 403, the light emitter 191L emits a red light to the entire left lateral surface 403.

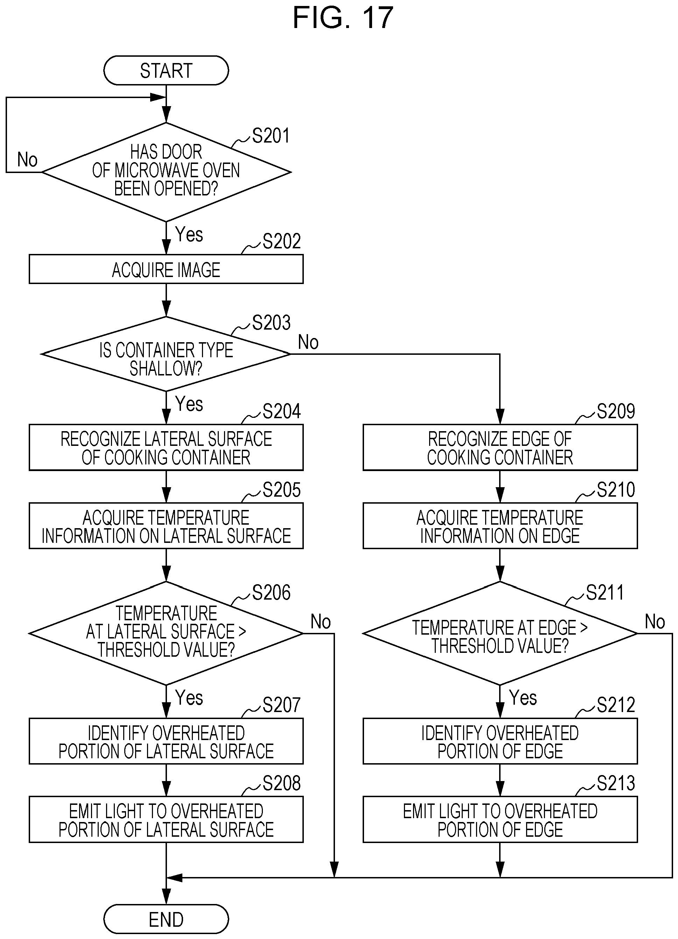

Fig. 17 is a flowchart illustrating the processing operation of the control device 110a.

First, the control device 110a determines whether or not cooking by the microwave oven 350 has been completed and the door of the microwave oven 350 is opened (step S201). Here, when it is determined that the door is opened (Yes in step S201), the image acquirer 111a of the control device 110a obtains an image from cameras 192R, 192U and 192L (step S202).

Subsequently, the recognizer 112a determines whether or not the container type of the heated cooking container is deep using the determination rule 116a based on the image obtained from the camera 192R or 192L (step S203).

Here, when it is determined that the container type of the cooking container deep (Yes in step S203), the recognizer 112a recognizes the right lateral surface and the left lateral surface of the cooking container from the image obtained from each of the cameras 192R and 192L (step S204).

Subsequently, the overheated portion identifier 114a obtains temperature information on the right lateral surface and the left lateral surface (that is, the lateral surface) from the respective infrared sensors 193R and 193L via the temperature information acquirer 113a (step S205).

The overheated portion then identifier 114a determines whether or not the temperature in the lateral surface exceeds a threshold value (step S206).

Here, when it is determined that the temperature in the lateral surface exceeds a threshold value (Yes in step S206), the overheated portion identification unit 114a identifies an overheated portion in the lateral surface (step S207). The emission controller 115a then transmits emission instruction information to at least one of the light emitters 191R and 191L. Thus, at least one of the light emitters 191R and 191L emits light to an overheated portion in the lateral surface of the cooking container (step S208).

On the other hand, when it is determined that the container type of the cooking container is not deep (No in step S203), the recognizer 112a recognizes the edge of the cooking container from the image obtained from the camera 192U (step S209).

Subsequently, the overheated portion identifier 114a obtains temperature information of the edge from the infrared sensor 193U via the temperature information acquirer 113a (step S210).

The overheated portion identifier 114a determines whether or not the temperature in the edge exceeds a threshold value (step S211).

Here, when it is determined that the temperature in the edge exceeds a threshold value (Yes in step S211), the overheated portion identification unit 114a identifies an overheated portion in the edge (step S212). The emission controller 115a transmits emission instruction information to the light emitter 191U. Thus, the light emitter 191U emits light to the overheated portion in the edge of the cooking container (step S213).

### [Summary and Effect of Second Embodiment]

In a second embodiment, the detection device includes not only the camera 192U and the infrared sensor 193U, but also the cameras 192R and 192L, and the infrared sensors 193R and 193L. The cameras 192R and 192L capture a range including a cooking container placed in the microwave oven 350 from the lateral side of the cooking container. The infrared sensors 193R and 193L detect the temperatures of portions in ranges captured by respective cameras 192R and 192L. Here, the camera 192R or 192L is a side camera, and the infrared sensor 193R or 193L is a side infrared sensor. The cooking support system 100a includes not only the light emitter 191U, but also the light emitters 191R and 192L that emit light to the cooking container from the lateral side of the cooking container. The light emitters 191R and 192L are side light emitters.

In the second embodiment, the control device 110a obtains detection information that includes an image (upper surface image) obtained by capturing with the camera 192U, an image (lateral surface image) obtained by capturing with the cameras 192R and 192L, temperature information (upper surface temperature information) obtained by detection with the infrared sensor 193U, and temperature information (lateral surface temperature information) obtained by detection with the infrared sensors 193R and 193L.

The control device 110a determines the type of the cooking container based on a side image.

Also, when an overheated portion is identified, the control device 110a selects a set consisting of the upper surface image and the upper surface temperature information or a set consisting of the lateral surface image and the lateral surface temperature information according to the determined container type, and identifies an overheated portion in the edge or the lateral surface of the cooking container based on the selected set. When light is to be emitted to the identified overheated portion in the lateral surface, the control device 110a transmits emission instruction information to at least one of the light emitters 191R and 191L instead of the light emitter 191U, thereby causing the at least one light emitter to emit light to the identified overheated portion.

A user puts a cooking container in the microwave oven 350 for cooking, and when the cooking container is taken out, the user holds the lateral surface of the cooking container or the edge of the cooking container according to the type of the cooking container. For instance, when the cooking container is a cup or a glass, a user tends to hold the lateral surface of the cooking container, and when the cooking container is a dish or the like a user tends to hold the edge of the cooking container. Therefore, in the second embodiment, an overheated portion in the edge or the lateral surface of the cooking container is identified according to the type of the cooking container and light is emitted to the overheated portion, and thus the risk of burn injuries can be reduced regardless of the type of the cooking container.

### [First Modification of Second Embodiment]

In the second embodiment, the cooking support system 100a emits light to an overheated portion in the right lateral surface 402 and the left lateral surface 403 (that is, the lateral surface) of the cooking container 400b, and does not emit light to any portion (that is, non-overheated portion) other than the overheated portion. In contrast, similarly to the second modification of the first embodiment, the cooking support system 100a in the first modification emits light of a color different from the color of the light emitted to the overheated portion to a non-overheated portion in the lateral surface.

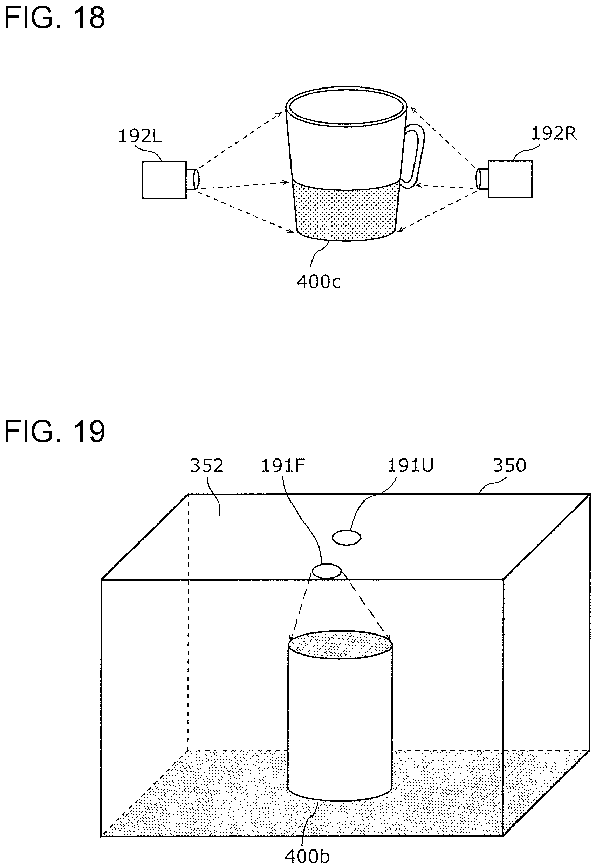

Fig. 18 illustrates an example of the lateral surface of a cooking container to which light is emitted by the light emitters 191R and 191L.

Upon obtaining identification area information on a cooking container 400c of the container type "deep", the emission controller 115a transmits emission instruction information to the light emitters 191R and 191L. The identification area information indicates the area of the lateral surface of the cooking container 400c, and the area of an overheated portion in the lateral surface. Also, the emission instruction information is information instructing that for instance, a red light is to be emitted to the area of the overheated portion indicated by the identification area information, and, for instance, a green light is to be emitted to the area other than the overheated portion out of the lateral surface indicated by the identification area information.

When receiving such emission designation information, as illustrated in Fig. 18, the light emitters 191R and 191L emit, for instance, a red light to an overheated portion at an upper portion of the lateral surface of the cooking container 400c, and emit, for instance, a green light to a non-overheated portion at a lower portion of the lateral surface of the cooking container 400c.

In this manner, in the first modification, when the light emitters 191R and 191L are caused to emit light, the control device 110a causes the light emitters 191R and 191L to emit light of a color different from the color of the light emitted to the overheated portion to any portion other than the overheated portion in the lateral surface.

This allows a user to be properly notified of the overheated portion in the lateral surface, as well as of the non-overheated portion (for instance, lower portion of the lateral surface) in the lateral surface. That is, a user can be properly notified of a portion which may be touched by a bare hand. Consequently, a user can save time and effort for preparing gloves such as a mitten.

### [Second Modification of Second Embodiment]

In a second embodiment, the cooking support system 100a includes two light emitters 191R and 191L that emit light to the lateral surface of a cooking container. In contrast, the cooking support system 100a in the second modification includes only one light emitter that emits light to the lateral surface of a cooking container.

Fig. 19 illustrates an example of the lateral surface of a cooking container to which light is emitted from an upper position.

The cooking support system 100a includes a light emitter 191F disposed in the upper wall 352 of the microwave oven 350 instead of the light emitters 191R and 191L. Specifically, the light emitter 191F is disposed on the door side (in other words, the user side or the near side) in the upper wall 352 of the microwave oven 350, and emits light from an upper diagonal position to the lateral surface (that is, the front surface) of the cooking container 400b.

Even with the cooking support system 100a including the light emitter 191F, the same effect as in the second embodiment is provided.

### (Third Embodiment)

In the first and second embodiments and their modifications, the cooking support system includes an infrared sensor, and the control device obtains temperature information indicating the temperature of each portion of the cooking container from the infrared sensor. In contrast, in this embodiment, the cooking support system does not include an infrared sensor, and the control device estimates the temperature of each portion of the cooking container.

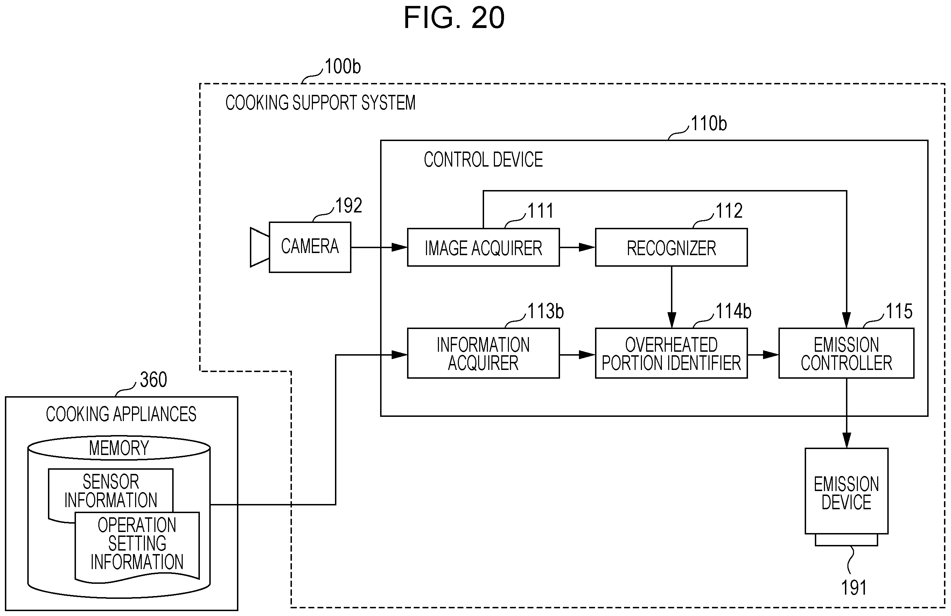

Fig. 20 is a block diagram illustrating an example of the configuration of the cooking support system in this embodiment.

A cooking support system 100b in this embodiment includes a control device 110b, a camera 192, and a light emitter 191, but does not include an infrared sensor. Similarly to the control device 110 in the first embodiment, the control device 110b includes the image acquirer 111, the recognizer 112, and the emission controller 115. In addition, the control device 110b includes an information acquirer 113b and an overheated portion identifier 114b instead of the temperature information acquirer 113 and the overheated portion identifier 114 in the first embodiment.

The information acquirer 113b obtains sensor information and operation setting information from a cooking device 360. Here, the sensor information and the operation setting information are temperature-related information related to temperatures of portions of a cooking container, and is stored in a memory of the cooking device 360. It is to be noted that the cooking device 360 may be the above-described IH cooking heater 300, may be the microwave oven 350, or may be another cooking device.

The sensor information indicates temperatures in part of a cooking container, detected by a sensor mounted in the cooking device 360. The sensor is a temperature sensor or an infrared sensor. For instance, when the cooking device 360 is an IH cooking heater, a sensor is mounted in the top plate of the IH cooking heater. Sensor information indicating the temperature at the bottom of the cooking container placed on the top plate is obtained by the sensor, and is stored in a memory of the cooking device 360.

The operation setting information is information that is set to the cooking device 360 for cooking using a cooking container. For instance, the operation setting information indicates elapsed time since the start of cooking with the cooking device 360 by an operation of a user, that is, elapsed time of heating to a cooking container. Alternatively, the operation setting information indicates a preset temperature of heating to a cooking container, which is set to the cooking device 360 by an operation of a user. Alternatively, the operation setting information indicates a mode (for instance, "deep fry" mode) of heating to a cooking container, which is set to the cooking device 360 by an operation of a user. The operation setting information may indicate all of or one of elapsed time of heating, preset temperature, and mode.

The overheated portion identifier 114b estimates the temperature of each portion of the edge of the cooking container recognized by the recognizer 112, based on the temperature-related information. For instance, when the temperature-related information includes the sensor information that indicates the temperature of the bottom of the cooking container, and the operation setting information that indicates elapsed time, the overheated portion identifier 114b estimates the temperature of each portion of the edge of the cooking container based on the sensor information and the operation setting information. More specifically, the overheated portion identifier 114b refers to a temperature characteristic table that indicates a relationship between temperatures of the bottom and the edge of the cooking container, and elapsed time. In other words, in the temperature characteristic table, the overheated portion identifier 114b finds a temperature of the edge which is associated with the temperature of the bottom of the cooking container indicated by the sensor information, and the elapsed time indicated by the operation setting information. Subsequently, the overheated portion identifier 114b calculates a temperature gradient of each portion of the edge according to the shape and size of the edge of the cooking container which have been recognized by the recognizer 112. The overheated portion identifier 114b then estimates the temperature of each portion of the edge based on the temperature found by the above-mentioned reference of data and the temperature gradient. For instance, the overheated portion identifier 114b calculates a temperature gradient in which the temperature at a position increases as the position is closer to the center of the edge and the temperature at a position decreases as the position is further away from the center. The overheated portion identifier 114b then estimates the temperature of each portion of the edge so that the average temperature of the edge equals to the temperature found by the above-mentioned reference of data. Although the operation setting information indicates lapsed time in the above-described example, the operation setting information may indicate preset temperature or mode. Even in this case, the overheated portion identifier 114b can estimate the temperature of each portion of the edge by referring to the temperature characteristic table that indicates the relationship between the bottom of the edge of a cooking container and the preset temperature or mode indicated by the operation setting information.

The overheated portion identifier 114b then identifies an overheated portion based on the temperature of each portion in the estimated edge.

In the example illustrated in Fig. 20, the cooking device 360 and the control device 110b are connected by a cable, however, may be connected wirelessly,

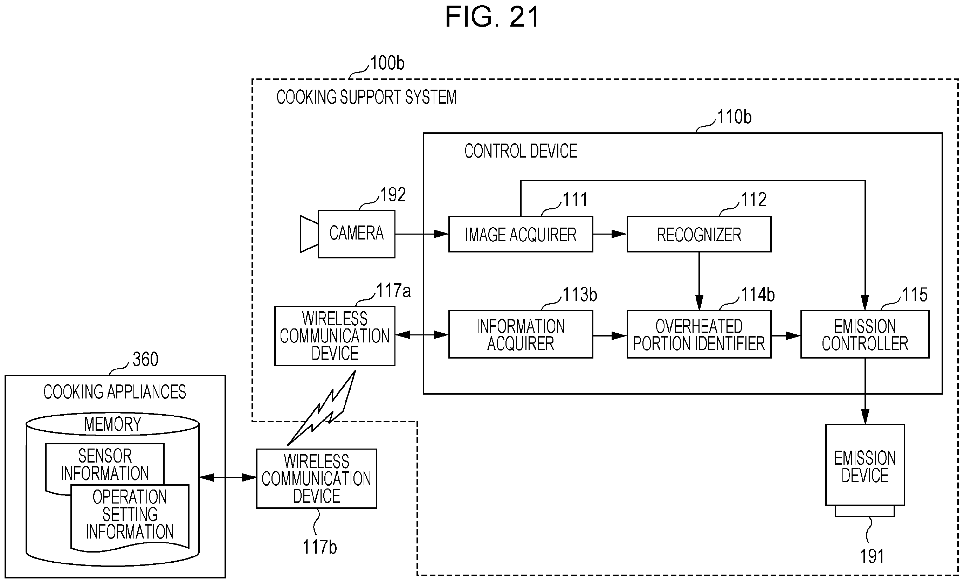

Fig. 21 is a block diagram illustrating another example of the configuration of the cooking support system 100b.

The cooking support system 100b in this embodiment may include a wireless communication device 117a as illustrated in Fig. 21. Here, the cooking device 360 is connected to a wireless communication device 117b that can wirelessly communicate with the wireless communication device 117a of the cooking support system 100b. Consequently, when the information acquirer 113b obtains sensor information and operation setting information from the cooking device 360, the information acquirer 113b requests transmission of those pieces of information to the cooking device 360, for instance, via the wireless communication devices 117a and 117b. The information acquirer 113b then obtains sensor information and operation setting information which are transmitted from the cooking device 360 via the wireless communication devices 117a and 117b.

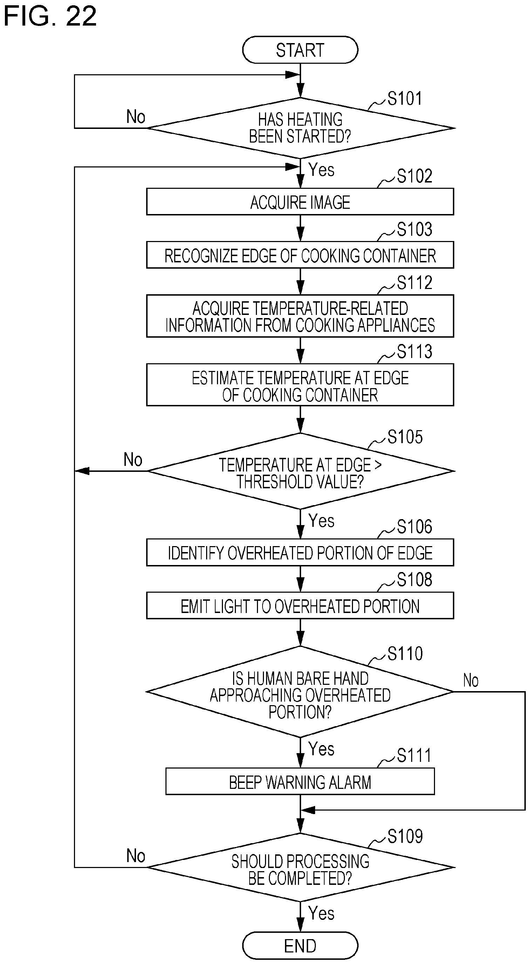

Fig. 22 is a flowchart illustrating the processing operation of the control device 110b of the cooking support system 100b in this embodiment.

Similarly to the flowchart illustrated in Fig. 9, the control device 110b in this embodiment performs the processing of steps S101 to S103, and the processing of steps S105 to S111. However, the control device 110b in this embodiment performs the processing of steps S112 and S113 instead of the processing of step S104 illustrated in Fig. 9.

Specifically, the information acquirer 113b of the control device 110b obtains temperature-related information from the cooking device 360 (step S112). Subsequently, the overheated portion identifier 114b estimates the temperature of each portion of the edge of the cooking container based on the temperature-related information (step S113).

Here, the recognizer 112 may identify the type of the cooking container from the image obtained by capturing with the camera 192. In this case, the information acquirer 113b obtains temperature-related information including type information that indicates the type of the cooking container. For instance, the type may be a model name of the cooking container, may be quality of material or raw material of the cooking container (stainless steel or enamel), or may be a physical property value such as the thermal conductivity of the cooking container. The recognizer 112 identifies the contour and size of a cooking container by performing edge detection to the above-mentioned image, for instance. Subsequently, the recognizer 112 refers to a container type table that for each cooking container, indicates the contour and size of the cooking container, and the type of the cooking container in association with each other. In the container type table, the recognizer 112 recognizes the type associated with the contour and size of the cooking container identified by edge detection, as the type of the cooking container captured by the camera 192. It is to be noted that the recognizer 112 may recognize the type of the cooking container based on the information from various sensors mounted in the cooking device 360.

The overheated portion identifier 114b estimates the temperature of each portion of the edge of the cooking container based on the temperature-related information including type information. Also at this point, the overheated portion identifier 114 may estimate the temperature by referring to the temperature characteristic table. For instance, the temperature characteristic table indicates, for each type of cooking container, a relationship between the type and temperatures of the bottom and the edge of the type of cooking container.

The overheated portion identifier 114b may first estimate the temperature of each portion of the edge of the cooking container without using the type of the cooking container, and subsequently, may correct the estimated temperature using the type of the cooking container. Specifically, the overheated portion identifier 114b corrects the estimated temperature by adding or subtracting a correction value according to the type of the cooking container to or from the estimated temperature.

The overheated portion identifier 114b can improve the accuracy of estimation of the temperature of each portion of the edge for increased amount of information included in the temperature-related information. Also, the temperature-related information may include only the sensor information, may include the sensor information and the operation setting information, or may include the sensor information and the type information. It is to be noted that a combination of the pieces of information included in the temperature-related information may be made in any manner.

As described above, in this embodiment, even though the cooking support system 100b does not include a detection device that detects at least the temperature of each portion of a cooking container, an overheated portion is identified by estimating the temperature of each portion in the edge of the cooking container. Consequently, similarly to the first and second embodiments and their modifications, it is possible to allow a user to be properly notified of the overheated portion in the edge which is likely to be held by the user in the cooking container.

### (Summary)

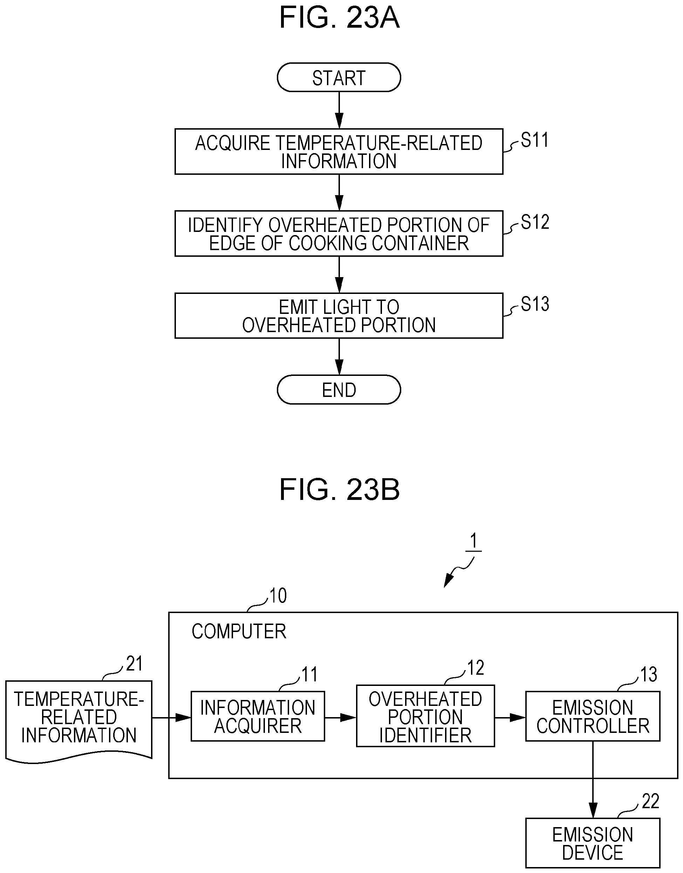

Fig. 23A is a flowchart of a cooking support method according to an aspect of the present disclosure.

A cooking support method according to an aspect of the present disclosure is a cooking support method in a cooking support system that supports cooking of a user. It is to be noted that the cooking support system has a light emitter that emits light and a computer. In the cooking support method, the computer obtains temperature-related information that is related to the temperature of each portion of a cooking container (step S11). Subsequently, the computer recognizes the edge of the cooking container placed on a cooking device that cooks by heating, and identifies an overheated portion which is a portion where the temperature exceeds a threshold value in the edge, based on the temperature-related information (step S12). The computer then transmits emission instruction information to the light emitter, thereby causing the light emitter to emit light to the identified overheated portion (step S13).

Fig. 23B illustrates the configuration of a cooking support system according to an aspect of the present disclosure.

A cooking supporting system 1 according to an aspect of the present disclosure is a cooking support system that supports cooking of a user, and includes a light emitter 22 that emits light, and a computer 10. The computer 10 includes an information acquirer 11, an overheated portion identifier 12, and an emission controller 13. The information acquirer 11 obtains temperature-related information related to the temperature of each portion of a cooking container. The overheated portion identifier 12 recognizes the edge of the cooking container placed on a cooking device that cooks by heating, and identifies an overheated portion which is a portion where the temperature exceeds a threshold value in the edge based on the temperature-related information. The emission controller 13 transmits emission instruction information to the light emitter 22, thereby causing the light emitter 22 to emit light to the identified overheated portion.

In the first and second embodiments and their modifications, the cooking support system 1 further has a detection device that detects at least the temperature of each portion of the cooking container. The computer 10 then obtains temperature-related information from the detection device, the temperature-related information being detection information indicating a result of the detection by the detection device, and recognizes the edge of the cooking container and identifies an overheated portion based on the detection information.

Thus, the edge of the cooking container is recognized, and light is emitted to an overheated portion in the edge. Therefore, it is possible to allow a user to be properly notified of the overheated portion in the edge which is likely to be held by the user in the cooking container. Consequently, it is possible to reduce the occurrence of burn injuries due to contact of a bare hand with an overheated portion. Specifically, it is possible to reduce the risk of burn injuries due to contact of a bare hand with a highly heated cooking container such as a dish or a pot. Also, since light is not emitted to the cooking ingredients in the cooking container, it is possible to reduce the effect on the visual determination of a user as to whether or not the cooking ingredients have been cooked well.

In the third embodiment, the cooking support system 1 further has a camera that captures a range including a cooking container placed on a cooking device. When the computer 10 identifies an overheated portion, an image obtained by capturing with the camera is obtained, the edge is recognized based on the image, and the temperature of each portion in the edge is estimated based on the temperature-related information described above. The computer 10 identifies an overheated portion based on the temperature of each portion in the estimated edge.

Thus, even when the cooking support system 1 is not equipped with a detection device that detects at least the temperature of each portion of the cooking container, the temperature of each portion in the edge of the cooking container is estimated, and an overheated portion is identified. Therefore, similarly to what has been described above, it is possible to allow a user to be properly notified of the overheated portion in the edge which is likely to be held by the user in the cooking container.

In the embodiments and their modifications, the control device may be formed of dedicated hardware or may be implemented by executing a software program. Here, a software program that implements the control device in the embodiments and their modifications causes a computer to execute the steps of the flowcharts illustrated in Figs. 8, 9, 17, 22 and 23A.

Although a cooking support method and a cooking support system according to one or more aspects have been described based on the embodiments and their modifications so far, the present disclosure is not limited to the embodiments and their modifications. An embodiment made by applying various alterations which may occur to those skilled in the art to the embodiments and their modifications, and an embodiment constructed by combining some components in different embodiments and modifications are also included in the scope of the present disclosure as long as not departing from the spirit of the present disclosure.

For instance, in the first and second embodiments and their modifications, the cooking support system includes a camera. However, the cooking support system may not include a camera. In this case, the cooking support system includes a thermal image sensor that is an infrared sensor as a detection device. When the control device identifies an overheated portion, the edge is recognized based on a thermal image which is detection information indicating a temperature distribution of a range including the cooking container, and an overheated portion is identified based on the temperature distribution in the recognized edge. It is to be noted that the thermal image is an image outputted from the thermal image sensor. When a cooking container is heated by a cooking device, the temperature of the cooking device becomes higher than the temperature of the surroundings of the cooking device. Therefore, similarly to an image of a camera, an image of the cooking container appears in the thermal image. The control device can recognize the edge or the lateral surface of the cooking container from the image of the cooking container that appears in the thermal image.

Although a red light is emitted to an overheated portion, and a green light is emitted to a non-overheated portion in the first and second embodiments and their modifications, the color of the light emitted to those portions may be changed according to the color of an overheated portion or a non-overheated portion in a cooking container.

For instance, the emission controller identifies the color of an overheated portion of the cooking container which appears in an image obtained by capturing with a camera, and causes the light emitter to emit a complementary color (that is, opposite color) of the color of the overheated portion. Thus, even when the overheated portion is red, it is possible to allow a user to be notified of the overheated portion in a more comprehensible manner.

Alternatively, the emission controller may blink light instead of changing the color of light to be emitted. Alternatively, the emission controller may output alarm sound from a speaker while emitting light. Even in this case, it is possible to allow a user to be notified of the overheated portion in a more comprehensible manner.

Although a red light is emitted to an overheated portion, and a green light is emitted to a non-overheated portion in the first and second embodiments and their modifications, light of a color according to the temperature of an overheated portion may be emitted to the overheated portion, and light of a color according to the temperature of a non-overheated may be emitted to the non-overheated portion. For instance, when the temperature of an overheated portion is greater than or equal to 100 °C, a red light is emitted to the overheated portion, and when the temperature of an overheated portion is 80 °C or greater and less than 100 °C, an orange light is emitted to the overheated portion. Also, when the temperature of a non-overheated portion is 40 °C or greater and less than 60 °C, a yellow light is emitted to the non-overheated portion, and when the temperature of a non-overheated portion is 20 °C or greater and less than 40 °C, a green light is emitted to the non-overheated portion. In the example described above, light of a color according to the temperature of each of an overheated portion and a non-overheated portion is emitted. However, light of a color according to the difference between the temperature of an overheated portion or a non-overheated portion, and the temperature of a bare hand of a user may be emitted.

In the first embodiment, light is emitted when a bare hand approaches an overheated portion. However, it is not necessary to emit light when a bare hand with a cooking tool such as chopsticks or a ladle approaches an overheated portion. For instance, the emission controller recognizes a bare hand which appears in an image obtained by capturing with a camera, and when a cooking tool is held by the bare hand, the emission controller suspends transmission of emission instruction information to the light emitter. When a user cooks using the cooking tool, a user is with sufficient caution against burn injuries, and so if light is emitted to the cooking container even in such a situation, the light may interface with cooking of a user. Therefore, when a cooking tool is already held by a user, cooking of a user can be supported by stopping emission of light, and the power consumption of the cooking support system can be reduced.

Although the portion to which light is emitted is switched between the edge and the lateral surface according to the type of a cooking container in the first and second embodiments and their modifications, the portion to which light is emitted may be switched according to the height of a user. That is, the angle of emission of light may be changed according to the eye level of a user.

For instance, the cooking support system includes a camera that captures the face or the like of a user. The control device recognizes the face or the eyes of the user from an image obtained with the camera, and identifies the height of the user. When the height of the user is higher than a threshold value, the control device causes the light emitter to emit light to an overheated portion in the edge of the cooking container, and When the height of the user is lower than or equal to the threshold value, the control device causes the light emitter to emit light to an overheated portion in the lateral surface of the cooking container. For instance, it may be assumed that when the position of the eyes of a user is sufficiently higher than the position of the cooking container, a user holds the edge of the cooking container, and when the position of the eyes of a user is not sufficiently higher than the position of the cooking container, a user holds the lateral surface of the cooking container. In such a case, it is possible to allow a user to be notified of the overheated portion in a more appropriate manner.

In the first and second embodiments and their modifications, an IH cooking heater and a microwave oven have been described as examples of a cooking device. However, the cooking device may be different from these devices. For instance, the cooking device may be a stove burner instead of an IH cooking heater.

The present disclosure allows a user to be properly notified of an overheated portion of a cooking container, and is applicable to a system that supports heat cooking with, for instance, an IH cooking heater or a microwave oven.

## Claims

1. A method comprising:
obtaining temperature-related information which is related to temperatures of portions of a cooking container (400), using a processor (190);
recognizing an edge of the cooking container (400) disposed in a cooking device that cooks by heating, using the processor,
**characterised by** identifying an overheated portion in the edge where a temperature exceeds a threshold value, based on the temperature-related information,
using the processor (190); transmitting emission instruction information to a light emitter (191) to cause the light emitter to emit light to the identified overheated portion, using the processor (190).

2. The method according to claim 1, further comprising
obtaining a result of detection by a sensor that detects at least the temperatures of the portions of the cooking container (400),
wherein, in the identifying,
the edge of the cooking container (400) is recognized, and the overheated portion is identified based on the result of detection.

3. The method according to claim 2, further comprising:
obtaining an image, by a camera (192), in a range including the cooking container (400) disposed in the cooking device,
wherein in the obtaining of the temperature information, the sensor is an infrared sensor (193),
obtaining temperature information indicating the temperatures of the portions in the range, by the infrared sensor (193), wherein, in the identifying,
the edge is recognized based on the image, and the overheating portion is identified based on the temperatures of the portions in the recognized edge out of the temperatures of the portions indicated by the temperature information.

4. The method according to claim 3,
wherein the camera (192) captures the range from a position above the cooking container (400), and
the light emitter (191), when receiving the emission instruction information, emits light to the overheated portion In the edge from a position above the cooking container (400).

5. The method according to claim 4, further comprising:
determining whether or not a bare human hand is approaching the overheated portion, based on the image; and
when the light emitter (191) is caused to emit light, transmitting the emission instruction information to the light emitter (191) at a timing at which it is determined that the bare human hand is approaching the overheated portion.

6. The method according to claim 5, further comprising:
determining whether or not a bare human hand is approaching the overheated portion based on the image obtained by the capturing with the camera (192) when light is emitted to the overheated portion by the light emitter (191); and
when it is determined that the bare human hand is approaching the overheated portion, performing processing to prompt warnings to people around the cooking device.

7. The method according to claim 6,
wherein when the light emitter is caused to emit light,
light of a color different from a color of the light emitted to the overheated portion is emitted to any portion other than the overheated portion in the edge.

8. The method according to claim 4, further comprising:
obtaining a lateral surface image by a side-camera (192L) from a lateral side of the cooking container (400) in the range;
obtaining lateral surface temperature information on portions captured by the side-camera (192L), by a side-infrared sensor (193L); and
determining a type of the cooking container (400) based on the lateral surface image,
wherein in the identifying,
a set consisting of the image and the temperature information or a set consisting of the lateral surface image and the lateral surface temperature information is selected according to the determined type, and the overheated portion in the edge or the lateral surface of the cooking container (400) is identified based on the selected set,
wherein in the emitting of the light,
when the overheated portion is in the lateral surface, light is emitted to the identified overheated portion by transmitting the emission instruction information to a side light emitter (191L) that emits light to the cooking container (400) from the lateral side of the cooking container (400), instead of to the light emitter.

9. The method according to claim 2,
wherein the sensor is a thermal image sensor, and
a thermal image indicating a temperature distribution in the range is obtained by the thermal image sensor,
wherein in the identifying,
the edge is recognized based on the thermal image, and the overheated portion is identified based on the temperature distribution in the recognized edge.

10. The method according to claim 1, further comprising:
obtaining an image, by a camera (192), in a range including the cooking container (400) placed in the cooking device,
wherein in the identifying,
the edge is recognized based on the image, and
temperatures of the portions in the edge are estimated based on the temperature-related information, and the overheated portion is identified based on the estimated temperatures of the portions in the edge.

11. The method according to claim 10,
wherein, the temperature-related information includes,
sensor information indicating part of temperatures of the cooking container (400) which are detected by a sensor mounted in the cooking device.

12. The method according to claim 11,
wherein the temperature-related information further includes operation setting information which is set to the cooking device for cooking using the cooking container (400).

13. The method according to claim 12,
wherein the operation setting information indicates at least one of a heating duration time, a set temperature and a mode for the cooking container (400).

14. The method according to claim 13,
wherein the temperature-related information further includes type information that indicates a type of the cooking container (400).

15. A system comprising:
a processor (190);
a light emitter (191); and
a memory storing thereon a computer program, which when executed by the processor (190), causes the processor (190) to perform operations including:
obtaining temperature-related information which is related to temperatures at portions of a cooking container (400);
recognizing an edge of the cooking container (400) disposed in a cooking device that cooks by heating;
identifying an overheated portion in the edge where a temperature exceeds a threshold value, based on the temperature-related information; and
transmitting emission instruction information to the light emitter (191) to cause the light emitter (191) to emit a light to the identified overheated portion.

## Patentansprüche

1. Verfahren, umfassend:
Erhalten von temperaturbezogenen Informationen, die sich auf Temperaturen von Teilen eines Kochbehälters (400) beziehen, unter Verwendung eines Prozessors (190);
Erkennen eines Randes des Kochbehälters (400), der in einer Kochvorrichtung angeordnet ist, die durch Erwärmen kocht, unter Verwendung des Prozessors, **gekennzeichnet durch**
Identifizieren eines überhitzten Randes, an dem die Temperatur einen Schwellenwert überschreitet, basierend auf den temperaturbezogenen Informationen, unter Verwendung des Prozessors (190);
Senden von Emissionsbefehlsinformationen an einen Lichtemitter (191), um den Lichtemitter dazu zu veranlassen, Licht zu dem identifizierten überhitzten Teil zu emittieren, unter Verwendung des Prozessors (190).

2. Verfahren nach Anspruch 1, des Weiteren umfassend:
Erhalten eines Ermittlungsergebnisse von einem Sensor, der mindestens die Temperaturen der Teile des Kochbehälters (400) ermittelt,
wobei, beim Identifizieren,
der Rand des Kochbehälters (400) erkannt wird und der überhitzte Teil basierend auf dem Ermittlungsergebnis identifiziert wird.

3. Verfahren nach Anspruch 2, des Weiteren umfassend:
Erhalten eines Bildes, von einer Kamera (192) in einem Bereich, der den in der Kochvorrichtung angeordneten Kochbehälter (400) einschließt,
wobei bei dem Erhalten der Temperaturinformationen der Sensor ein Infrarotsensor (193) ist,
Erhalten von Temperaturinformationen, welche die Temperaturen der Teile in dem Bereich anzeigen, durch den Infrarotsensor (193),
wobei, beim Identifizieren,
der Rand basierend auf dem Bild erkannt wird, und der Überhitzungsteil basierend auf den Temperaturen der Teile in dem erkannten Rand aus den Temperaturen der Teile identifiziert wird, die durch die Temperaturinformationen angezeigt werden.

4. Verfahren nach Anspruch 3,
wobei die Kamera (192) den Bereich von einer Position oberhalb des Kochbehälters (400) aufnimmt, und
der Lichtemitter (191) beim Empfangen der Emissionsbefehlsinformationen Licht zu dem überhitzten Teil am Rand von einer Position über dem Kochbehälter (400) emittiert.

5. Verfahren nach Anspruch 4, des Weiteren umfassend:
Bestimmen, ob sich eine ungeschützte menschliche Hand dem überhitzten Teil nähert, basierend auf dem Bild; und
wenn der Lichtemitter (141) zum Emittieren von Licht veranlasst wird, Senden der Emissionsbefehlsinformationen an den Lichtemitter (191) zu einem Zeitpunkt, an dem bestimmt wird, dass sich die ungeschützte menschliche Hand dem überhitzten Teil nähert.

6. Verfahren nach Anspruch 5, des Weiteren umfassend:
Bestimmen, ob sich eine ungeschützte menschliche Hand dem überhitzten Teil nähert, basierend auf dem Bild, das durch die Aufnahme mit der Kamera (192) erhalten wird, wenn Licht zu dem überhitzten Teil durch den Lichtemitter (191) emittiert wird; und
wenn bestimmt wird, dass sich die ungeschützte menschliche Hand dem überhitzten Teil nähert, Durchführen von Verarbeitung, um Warnungen an Menschen rund um die Kochvorrichtung auszugeben.

7. Verfahren nach Anspruch 6,
wobei, wenn der Lichtemitter zum Emittieren von Licht veranlasst wird,
Licht mit einer anderen Farbe als das zu dem überhitzten Teil emittierte Licht zu einem anderen Teil als dem überhitzten Teil am Rand emittiert wird.

8. Verfahren nach Anspruch 4, des Weiteren umfassend:
Erhalten eines lateralen Oberflächenbildes durch eine Seitenkamera (192L) von einer lateralen Seite des Kochbehälters (400) in dem Bereich;
Erhalten von lateralen Oberflächentemperaturinformationen an Teilen, die von der Seitenkamera (192L) aufgenommen werden, durch einen seitlichen Infrarotsensor (193L);
Bestimmen eines Typs des Kochbehälters (400) basierend auf dem lateralen Oberflächenbild,
wobei, beim Identifizieren,
ein Satz, der aus dem Bild und den Temperaturinformationen besteht, oder ein Satz, der aus dem lateralen Oberflächenbild und den lateralen Oberflächentemperaturinformationen besteht, entsprechend dem bestimmten Typ ausgewählt wird, und der überhitzte Teil am Rand oder der lateralen Oberfläche des Kochbehälters (400) basierend auf dem ausgewählten Satz identifiziert wird,
wobei, wenn sich beim Emittieren des Lichts der überhitzte Teil in der lateralen Oberfläche befindet, Licht zu dem identifizierten überhitzten Teil durch Senden der Emissionsbefehlsinformationen an einen seitlichen Lichtemitter (191L), der Licht zu dem Kochbehälter (400) von der lateralen Seite des Kochbehälters (400) aus emittiert, anstelle des Lichtemitters emittiert wird.

9. Verfahren nach Anspruch 2,
wobei der Sensor ein Wärmebildsensor ist, und
ein Wärmebild, welches eine Temperaturverteilung in dem Bereich anzeigt, von dem Wärmebildsensor erhalten wird,
wobei, beim Identifizieren,
der Rand basierend auf dem Wärmebild erkannt wird, und der überhitzte Teil basierend auf der Temperaturverteilung in dem erkannten Rand identifiziert wird.

10. Verfahren nach Anspruch 1, des Weiteren umfassend:
Erhalten eines Bildes, von einer Kamera (192) in einem Bereich, der den in der Kochvorrichtung platzierten Kochbehälter (400) einschließt,
wobei, beim Identifizieren,
der Rand basierend auf dem Bild erkannt wird, und
Temperaturen der Teile am Rand basierend auf den temperaturbezogenen Informationen geschätzt werden, und der überhitzte Teil basierend auf den geschätzten Temperaturen der Teile am Rand identifiziert wird.

11. Verfahren nach Anspruch 10,
wobei die temperaturbezogenen Informationen Folgendes beinhalten,
Sensorinformationen, die einen Teil der Temperaturen des Kochbehälters (400) anzeigen, die von einem Sensor ermittelt werden, der in der Kochvorrichtung montiert ist.

12. Verfahren nach Anspruch 11,
wobei die temperaturbezogenen Informationen des Weiteren Betriebseinstellungsinformationen beinhalten, die an der Kochvorrichtung zum Kochen unter Verwendung des Kochbehälters (400) eingestellt werden.

13. Verfahren nach Anspruch 12,
wobei die Betriebseinstellungsinformationen mindestens eines aus einer Heizdauer, einer eingestellten Temperatur und einem Modus für den Kochbehälter (400) anzeigen.

14. Verfahren nach Anspruch 13,
wobei die temperaturbezogenen Informationen des Weiteren Typinformationen beinhalten, die einen Typ des Kochbehälters (400) anzeigen.

15. System, umfassend:
einen Prozessor (190);
einen Lichtemitter (191);
einen Speicher, auf dem ein Computerprogramm gespeichert ist, das bei Ausführung durch den Prozessor (190) den Prozessor (190) dazu veranlasst, Vorgänge durchzuführen, die beinhalten:
Erhalten von temperaturbezogenen Informationen, die sich auf Temperaturen an Teilen eines Kochbehälters (400) beziehen;
Erkennen eines Randes des Kochbehälters (400), der in einer Kochvorrichtung angeordnet ist, die durch Erwärmen kocht,
Identifizieren eines überhitzten Randes, an dem die Temperatur einen Schwellenwert überschreitet, basierend auf den temperaturbezogenen Informationen; und
Senden von Emissionsbefehlsinformationen an den Lichtemitter (191), um den Lichtemitter dazu zu veranlassen, Licht zu dem identifizierten überhitzten Teil zu emittieren.

## Revendications

1. Procédé comprenant :
la récupération d'informations liées à la température, lesquelles sont liées aux températures de parties d'un récipient de cuisson (400), en utilisant un processeur (190),
la reconnaissance du bord du récipient de cuisson (400) disposé dans un dispositif de cuisson qui cuit par chauffage, en utilisant le processeur, **caractérisé par**,
l'identification d'une partie surchauffée sur le bord où la température dépasse une valeur de seuil, sur la base des informations liées à la température, en utilisant le processeur (190),
la transmission d'informations d'instruction d'émission à un émetteur de lumière (191) afin d'amener l'émetteur de lumière à émettre de la lumière vers la partie surchauffée identifiée, en utilisant le processeur (190).

2. Procédé selon la revendication 1, comprenant en outre :
la récupération du résultat de la détection par un capteur qui détecte les températures des parties du récipient de cuisson (400),
dans lequel, lors de l'identification,
le bord du récipient de cuisson (400) est reconnu et la partie surchauffée est identifiée sur la base du résultat de la détection.

3. Procédé selon la revendication 2, comprenant en outre :
la récupération d'une image, grâce à un appareil de prise de vues (192), dans un champ incluant le récipient de cuisson (400) placé dans le dispositif de cuisson,
dans lequel, lors de la récupération des informations de température, le capteur est un capteur infrarouge (193),
la récupération d'informations de température indiquant les températures des parties dans le champ, grâce au capteur infrarouge (193),
dans lequel, lors de l'identification,
le bord est reconnu sur la base de l'image et la partie en surchauffe est identifiée sur la base des températures des parties situées sur le bord reconnu parmi les températures des parties indiquées par les informations de température.

4. Procédé selon la revendication 3,
dans lequel l'appareil de prise de vues (192) prend le champ depuis une position située au-dessus du récipient de cuisson (400), et
l'émetteur de lumière (191), lorsqu'il reçoit des informations d'instruction d'émission, émet la lumière vers la partie surchauffée sur le bord depuis une position située au-dessus du récipient de cuisson (400).

5. Procédé selon la revendication 4, comprenant en outre :
la détermination de ce qu'une main humaine nue se rapproche ou non de la partie surchauffée, sur la base de l'image, et
lorsque l'émetteur de lumière (191) est amené à émettre de la lumière, la transmission des informations d'instruction d'émission à l'émetteur de lumière (191) avec un séquencement auquel il est déterminé que la main humaine nue se rapproche de la partie surchauffée.

6. Procédé selon la revendication 5, comprenant en outre :
la détermination de ce qu'une main humaine nue se rapproche ou non de la partie surchauffée sur la base de l'image obtenue par la capture réalisée par l'appareil de prise de vues (192) lorsque la lumière est émise par l'émetteur de lumière (191) vers la partie surchauffée, et
lorsqu'il est déterminé que la main humaine nue se rapproche de la partie surchauffée, l'exécution d'un traitement pour émettre des alarmes vers les personnes autour du dispositif de cuisson.

7. Procédé selon la revendication 6,
dans lequel, lorsque l'émetteur de lumière est amené à émettre de la lumière,
de la lumière d'une couleur différente de la couleur de la lumière émise vers la partie surchauffée est émise vers toute partie différente de la partie surchauffée sur le bord.

8. Procédé selon la revendication 4, comprenant en outre :
la récupération d'une image de surface latérale par un appareil de prise de vues (192L) latéral depuis un côté latéral du récipient de cuisson (400) sur le bord,
la récupération d'informations sur la température de surface latérale sur des parties capturées par l'appareil de prise de vues (192L) latéral, grâce au capteur infrarouge (193L) latéral, et
la détermination du type de récipient de cuisson (400) sur la base de l'image de surface latérale,
dans lequel, lors de l'identification, un ensemble constitué de l'image et des informations de température, ou bien un ensemble constitué de l'image de surface latérale et des informations de température de surface latérale est sélectionné en fonction du type déterminé, et la partie surchauffée sur le bord ou sur la surface latérale du récipient de cuisson (400) est identifiée sur la base de l'ensemble sélectionné,
dans lequel, lors de l'émission de la lumière,
lorsque la partie surchauffée se trouve sur la surface latérale, de la lumière est émise vers la partie surchauffée identifiée en transmettant les informations d'instruction d'émission à l'émetteur de lumière (191L) latéral qui émet de la lumière vers le récipient de cuisson (400) depuis le flanc latéral du récipient de cuisson (400) à la place de l'émetteur de lumière.

9. Procédé selon la revendication 2,
dans lequel le capteur est un capteur d'image thermique, et
une image thermique indiquant la répartition de températures sur le bord est obtenue par le capteur d'image thermique,
dans lequel, lors de l'identification,
le bord est reconnu sur la base de l'image thermique et la partie surchauffée est identifiée sur la base de la répartition de températures sur le bord reconnu.

10. Procédé selon la revendication 1, comprenant en outre :
la récupération d'une image grâce à un appareil de prise de vues (192) placé dans le dispositif de cuisson,
dans lequel, lors de l'identification,
le bord est reconnu sur la base de l'image, et
des températures des parties sur le bord sont estimées sur la base des informations liées à la température, et la partie surchauffée est identifiée sur la base des températures estimées des parties sur le bord.

11. Procédé selon la revendication 10,
dans lequel les informations liées à la température incluent :
les informations de capteur indiquant une partie des températures du récipient de cuisson (400) qui sont détectées par un capteur monté dans le dispositif de cuisson.

12. Procédé selon la revendication 11,
dans lequel les informations liées à la température incluent en outre :
des informations de réglage du fonctionnement qui est pratiqué sur le dispositif de cuisson pour la cuisson en utilisant le récipient de cuisson (400).

13. Procédé selon la revendication 12,
dans lequel les informations de réglage de fonctionnement indiquent au moins l'un d'une durée de chauffage, d'une température réglée et d'un mode pour le récipient de cuisson (400).

14. Procédé selon la revendication 13,
dans lequel les informations liées à la température incluent en outre des informations de type qui indiquent le type du récipient de cuisson (400).

15. Système comprenant :
un processeur (190),
un émetteur de lumière (191), et
une mémoire stockant un programme informatique qui, lorsqu'il est exécuté par le processeur (190) amène le processeur (190) à réaliser des opérations incluant :
la récupération d'informations liées à la température, lesquelles sont liées aux températures localisées à des parties d'un récipient de cuisson (400),
la reconnaissance d'un bord du récipient de cuisson (400) disposé dans un dispositif de cuisson qui cuit par chauffage,
l'identification d'une partie surchauffée sur le bord où la température dépasse une valeur de seuil, sur la base des informations liées à la température, et
la transmission d'informations d'instruction d'émission à l'émetteur de lumière (191) afin d'amener l'émetteur de lumière (191) à émettre une lumière vers la partie surchauffée identifiée.
